# EUROPEAN PATENT SPECIFICATION

(11) **EP 2 412 811 B1**
(45) Date of publication and mention of the grant of the patent: **05.08.2015**
(21) Application number: 10756204.3
(22) Date of filing: 26.03.2010
(51) Int. Cl.: C12N 15/09, A61K 39/00, A61K 39/39, A61K 48/00, A61P 25/28, A61P 37/04

(54) **DNA VACCINE FOR ALZHEIMER'S DISEASE**
DNA-IMPFSTOFF FÜR MORBUS ALZHEIMER
VACCIN À ADN POUR MALADIE D'ALZHEIMER

(30) Priority: 26.03.2009 JP 2009075832
(43) Date of publication of application: 01.02.2012
(73) Proprietor: Matsumoto, Yoh, Saitama 336-0021 (JP)
(72) Inventor: Matsumoto, Yoh, Saitama 336-0021 (JP)
(74) Representative: Bohest AG
(86) International application number: PCT/JP2010/055308
(87) International publication number: WO 2010/110408

(56) References cited:
- WO-A2-2005/014041
- WO-A2-2008/097927
- WO-A2-2009/029272
- NINA MOVSESYAN ET AL: "Reducing AD-Like Pathology in 3xTg-AD Mouse Model by DNA Epitope Vaccine A Novel Immunotherapeutic Strategy", PLOS ONE, PUBLIC LIBRARY OF SCIENCE, SAN FRANCISCO, CA; US, vol. 3, no. 5, 1 May 2008 (2008-05-01), pages 1-13, XP007906459, ISSN: 1932-6203
- MATSUMOTO Y ET AL: "Development of a new DNA vaccine for Alzheimer disease targeting Abeta species and amyloiditogenic peptides in the brain", BRAIN PATHOLOGY, vol. 20, no. Suppl. 1, September 2010 (2010-09), page 16, XP009163728, & 17TH INTERNATIONAL CONGRESS OF NEUROPATHOLOGY (ICN 2010); SALZBURG, AUSTRIA; SEPTEMBER 11 -15, 2010 ISSN: 1015-6305
- OKURA YOSHIO ET AL: "Recent advance in immunotherapies for Alzheimer disease With special reference to DNA vaccination", HUMAN VACCINES, vol. 5, no. 6, June 2009 (2009-06), pages 373-380, XP002685287, ISSN: 1554-8619
- GHOCHIKYAN, A. ET AL.: 'Generation and characterization of the humoral immune response to DNA immunization with a chimeric -beta-amyloid- interleukin-4 minigene.' EUR. J. IMMUNOL. vol. 33, no. 12, 2003, pages 3232 - 3241, XP002315950
- ZOU, J. ET AL.: 'Vaccination of Alzheimer's model mice with adenovirus vector containing quadrivalent foldable Abeta1-15 reduce Abeta burden and behavioral impairment without Abeta-specific T cell response.' J. NEUROL. SCI. vol. 272, no. 1-2, 2008, pages 87 - 98, XP023520248
- OKURA, Y. ET AL.: 'Nonviral Ap DNA vaccine therapy against Alzheimer's disease: Long-term effects and safety.' PROC. NATL. ACAD. SCI. U.S.A. vol. 103, no. 25, 2006, pages 9619 - 9624, XP055019122
- OKURA, Y. ET AL.: 'Development of anti-Abeta vaccination as a promising therapy for Alzheimer's disease.' DRUG NEWS PERSPECT. vol. 20, no. 6, 2007, pages 379 - 386, XP001526078
- OKURA, Y. ET AL.: 'DNA vaccine therapy for Alzheimer's disease: Present status and future direction.' REJUVENATION RES. vol. 11, no. 2, 2008, pages 301 - 308, XP009163711
- YOSHIO OKURA ET AL.: 'Alzheimer's Disease ni Taisuru Shin Vaccine Ryoho - Genjo to Wareware no Kokoromi' BRAIN AND NERVE vol. 60, no. 8, 2008, pages 931 - 940, XP009156459
- OKURA, Y. ET AL.: 'Nonviral DNA vaccination augments microglial phagocytosis of beta-amyloid deposits as a major clearance pathway in an Alzheimer disease mouse model.' J. NEUROPATHOL. EXP. NEUROL. vol. 67, no. 11, 2008, pages 1063 - 1071, XP009163712
- YORIKO TOKITA ET AL.: 'Akagezaru o Mochiita Alzehimer's Disease ni Taisuru Hi Virus-sei DNA Vaccine no Koka to Anzensei Shiken' PROCEEDINGS OF THE JAPANESE SOCIETY FOR IMMUNOLOGY vol. 38, 2008, page 146, XP008167527

## Description

### TECHNICAL FIELD

The present invention relates to DNA vaccines for Alzheimer's disease.

### BACKGROUND ART

Alzheimer's disease (AD) is a disease which frequently occurs in middle-aged or older people and whose major symptom is cognitive impairment (including memory disorder, orientation disturbance, learning disorder, attention disorder, spatial cognitive impairment, problem-solving impairment, etc.). Symptoms of this disease progress over several years, during which there often appear problem behaviors such as persecutory delusion, hallucination, offensive language, violence, wandering, and unclean behavior. This causes unwanted effects not only on patients themselves, but also on those around them including their family members and physicians, nurses, therapists, etc.

Alzheimer's disease has three pathological features, i.e., senile plaques (Aβ deposition), neurofibrillary tangles (tau deposition), and neuronal loss. Many studies have indicated that Aβ deposition occurs prior to tau deposition and neuronal changes, and the "amyloid hypothesis" has become widely known, which states that prevention of Aβ deposition would allow some avoidance of the subsequent events, i.e., tau accumulation in neurons, loss of neurons and so on.

Based on the amyloid hypothesis, Aβ peptide vaccine therapy has been proposed as a radical therapy for Alzheimer's disease (Non-patent Document 1). This vaccine therapy is based on the experiments in mice, in which the mice were externally administered with the Aβ peptide together with an immune activator (adjuvant) to induce in vivo production of anti-Aβ antibody to thereby reduce Aβ accumulation in the brain.

However, clinical trials in humans have been discontinued because of side effect problems, such as meningoencephalitis developed in cases receiving real drugs.

Following these clinical trials, further development has proceeded in an attempt to design vaccine formulations which do not cause encephalitis. The Aβ peptide is known to induce Th1 responses primarily through its C-terminal fragment and Th2 responses through its N-terminal fragment. Thus, clinical trials have been conducted for vaccines comprising an N-terminal fragment of Aβ attached to a carrier protein (Non-patent Document 2).

However, it was still difficult to overcome side effects including encephalitis.

For this reason, DNA vaccine therapy has been developed as a new vaccine therapy for Alzheimer's disease, which is an alternative to these Aβ peptide vaccines. Moreover, the inventors of the present invention have developed DNA vaccines which are effective for elimination of deposited Aβ and are also highly safe (Non-patent Document 3). A known example of the DNA vaccines developed by the inventors is an IgL-Aβ-Fc vaccine (Patent Document 1).

Recent studies have been indicating that there are various subspecies of neurotoxic Aβ. Aβ oligomers, which are most intensively analyzed, can be divided into two major types, i.e., those of low-molecular-weight type assembled from 2, 3 or 4 molecules and those of high-molecular-weight type assembled from 12 or more molecules. Moreover, strong neurotoxicity is also observed in pEAβ3-42, which is N-terminally truncated and pyrrole-derivatized by post-translational modification (Non-patent Documents 4 and 5). Furthermore, some findings are also being obtained, which suggest that other molecules such as ABri (Non-patent Document 6) and ADan, which have high amyloid aggregation propensity although their amino acid sequences are completely different from that of Aβ, are also involved in the onset of Alzheimer's disease.

### Prior Art Documents

### Patent Documents

[Patent Document 1] WO 2005/014041 A2

### Non-patent Documents

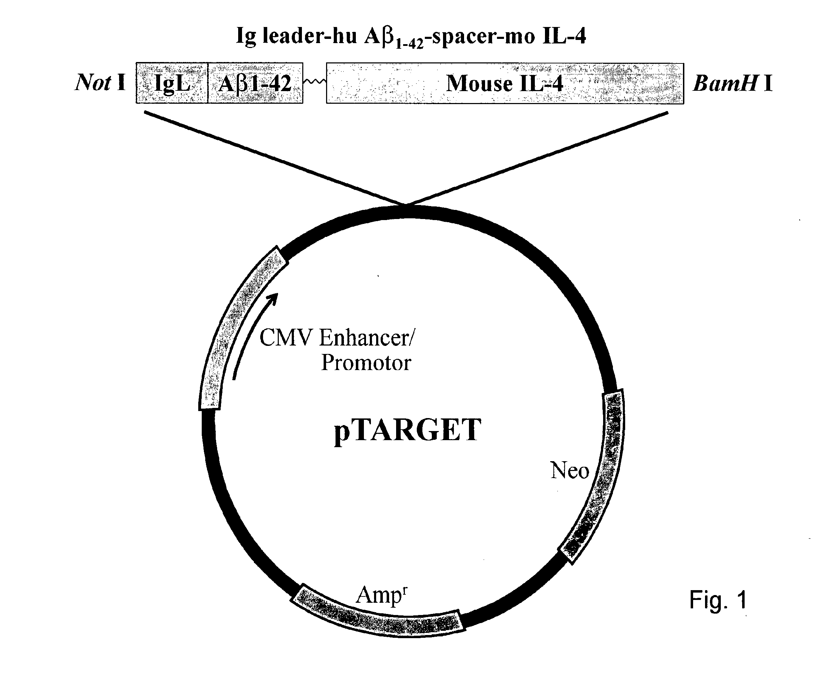

[Non-patent Document 1] Schenk D. et al., Nature 400: 173-177, 1999
[Non-patent Document 2] Masters CL et al., Brain 129: 2823-2839, 2006
[Non-patent Document 3] Y. Okura, et al., PNAS vol. 103: 9619-9624, 2006
[Non-patent Document 4] Saido et al., Neurosci Lett, 215, 173-176, 1996
[Non-patent Document 5] Schlenzig et al., Biochemistry, 48, 7072-7078, 2009
[Non-patent Document 6] Ghiso et al., Brain Pathol, 16, 71-79, 2006
[Non-patent Document 7] Okura et al., Proc. Natl. Acad. Sci. USA 103(25), 9619-9624, 2006 discloses in its Figure 1 a recombinant vector comprising Ig leader sequence, a single copy of Aβ1-42 gene, and human IgFc.

### DISCLOSURE OF THE INVENTION

### PROBLEMS TO BE SOLVED BY THE INVENTION

In light of these previous studies, there has been a demand for the development of DNA vaccine therapy which is more effective and safer than existing DNA vaccines.

### MEANS FOR SOLVING THE PROBLEMS

As a result of extensive and intensive efforts made to solve the above problems, the inventors of the present invention have constructed a vector which carries repeats of DNA encoding Aβ, DNA encoding a Th2 cytokine and DNA encoding an immunoglobulin Fc sequence, and have found that this vector has a therapeutic effect when administered to model animals of Alzheimer's disease. The inventors have further found that the above vector also induces antibodies against a wide variety of molecules with neurotoxicity and high amyloid aggregation propensity (e.g., pEAβ3-42, ABri, ADan). These findings led to the completion of the present invention.

Namely, the present invention is as follows.
(1) A recombinant vector, which comprises repeats of DNA encoding Aβ1-42, an immunoglobulin Fc sequence and DNA encoding interleukin-4.
(2) The recombinant vector according to (1) above, which comprises 2 to 4 repeats of DNA encoding Aβ1-42.
(3) The vector according to (2) above, wherein the immunoglobulin Fc sequence is human and the DNA encoding interleukin-4 is human.
(4) A DNA vaccine which comprises the recombinant vector according to any one of (1) to (3) above.
(5) The DNA vaccine according to (4) above, for use in the prevention or treatment of Alzheimer's disease.
(6) An inducer of anti-Aβ antibody, which comprises the recombinant vector according to (1) above.

### ADVANTAGEOUS EFFECT OF THE INVENTION

The present invention provides DNA vaccines for Alzheimer's disease. The DNA vaccines of the present invention can be regarded as having a high therapeutic effect on Alzheimer's disease, due to their high affinity for senile plaques. Moreover, the present invention allows induction of antibodies against a wide variety of molecules with neurotoxicity and high amyloid aggregation propensity (e.g., pEAβ3-42, ABri, ADan).

### BRIEF DESCRIPTION OF THE DRAWINGS

Figure 1 shows the construction of a vector for use as a reference DNA vaccine.
Figure 2 shows the construction of a vector for use as a reference DNA vaccine.
Figure 3 shows the Aβ-eliminating effect produced by reference DNA vaccines upon administration to Alzheimer's disease model mice.
Figure 4 shows increased levels of antibody with high affinity for Aβ deposition.
Figure 5 shows the construction of a vector for use as a reference DNA vaccine.
Figure 6 shows the construction of a vector for use as a reference DNA vaccine.
Figure 7 shows the construction of a vector for use as the DNA vaccine of the present invention.
Figure 8 shows the construction of a vector for use as the DNA vaccine of the present invention.
Figure 9 shows the construction of a vector for use as a reference DNA vaccine.
Figure 10 shows the construction of a vector for use as a reference DNA vaccine.
Figure 11 shows the results obtained for the amount of Aβ produced in cultured cells transfected with reference vaccines and the amount of Aβ released into the extracellular environment.
Figure 12 shows the anti-Aβ antibody-inducing effect in plasma produced by a reference vaccine and the vaccine of the present invention.
Figure 13 shows the brain Aβ-eliminating effect produced by the vaccine of the present invention.
Figure 14 shows the schedule of DNA vaccination and procedures for antibody titer assay.
Figure 15 shows the anti-Aβ1-42 antibody-inducing effect produced by YM3711 in rabbits.
Figure 16 shows the time course of anti-Aβ1-42 antibody induction mediated by YM3711 in rabbits.
Figure 17 shows the anti-Aβ1-42 antibody-inducing effect produced by YM3711 in wild-type mice and in disease model mice.
Figure 18 shows the anti-Aβ1-42 antibody-inducing effect produced by YM3711 in monkeys.
Figure 19 shows the time course of anti-Aβ1-42 antibody induction in monkeys after immunization with YM3711.
Figure 20 shows the anti-pEAb3-42 antibody-inducing effect produced by YM3711 in rabbits.
Figure 21 shows the anti-ABri antibody-inducing effect produced by YM3711 in rabbits.
Figure 22 shows the anti-ADan antibody-inducing effect produced by YM3711 in rabbits.
Figure 23 shows the anti-pEAb3-42 antibody-inducing effect produced by YM3711 in monkeys.
Figure 24 shows the anti-ADan antibody-inducing effect produced by YM3711 in monkeys.

### BEST MODES FOR CARRYING OUT THE INVENTION

The present invention will be described in more detail below. The following embodiments are illustrated to describe the present invention, and it is not intended to limit the present invention only to these embodiments.

The present invention is directed to a DNA vaccine for Alzheimer's disease. The present invention is also directed to a vector constructed by ligating the Aβ gene with the Th2 cytokine (IL-4) gene and by ligating the above vector with an immunoglobulin Fc (IgFc) sequence.

Such a DNA vaccine is intended to cause Aβ protein production in cells into which the vaccine is incorporated, which is accomplished when a gene for Aβ protein production is integrated into a vector (plasmid or virus) for gene transfer and administered to a patient. Aβ thus produced stimulates the immune system and induces anti-Aβ antibody (Y. Okura, et al., BRAIN and NERVE 60(8): 931-940, 2008). DNA vaccines remain in the body for a long time after administration and continue to slowly produce the encoded peptides, so that excessive immune responses can be avoided, and DNA vaccines can also be modified due to their simple structure (Tang DC et al., Nature 356: 152-154, 1992; Barry MA et al., Nature 377: 632-635, 1995). Moreover, they are advantageous in that immune responses induced in a host are of Th2 type (Tang DC et al., Nature 356: 152-154, 1992; Ulmer JB et al., Science 259: 1745-1749, 1993; Hoffman SL et al., Ann N Y Acad Sci 772: 88-94, 1995).

In view of these facts, the inventors of the present invention have conceived that a gene encoding the Th2 cytokine IL-4 is introduced together with the Aβ gene to thereby suppress side effects caused by cellular immunity, and have also constructed actual vaccines for Alzheimer's disease to study their effects. As a result, the vector of the present invention showed a high Aβ-eliminating effect at low doses and at reduced frequency. Moreover, the inventors have succeeded in promoting the extracellular release of expressed Aβ to more strongly stimulate immune responses against Aβ by introduction of a gene encoding an IgFc sequence, in addition to the genes encoding Aβ and the Th2 cytokine IL-4. Further, the inventors have succeeded in developing a vaccine with a higher brain Aβ-eliminating effect by construction of a vector comprising repeats of Aβ-encoding DNA. Furthermore, the inventors have found that the above vector also induces antibodies against a wide variety of molecules with neurotoxicity and high amyloid aggregation propensity (e.g., pEAβ3-42, ABri, ADan).

Thus, the vector of the present invention comprises DNA encoding an IgFc sequence, in addition to the DNA encoding Aβ and the DNA encoding a Th2 cytokine. The DNA encoding Aβ contained in the vector of the present invention consists of repeats of itself.

Although such Aβ, Th2 cytokine and IgFc sequence may be derived from animals of the same or different species as the target animal to be administered with the DNA vaccine, those derived from animals of the same species are preferred for use.

DNA encoding Aβ, DNA encoding a Th2 cytokine and DNA encoding an IgFc sequence have already been cloned. Thus, DNAs contained in the vector of the present invention can be obtained by standard genetic engineering procedures. For example, it is possible to use nucleic acid synthesis techniques with a DNA synthesizer, which are commonly used as genetic engineering procedures. Alternatively, it is also possible to use PCR techniques in which DNA sequences serving as templates are isolated or synthesized, and primers specific for each DNA are then designed to amplify each gene sequence with a PCR system, or to use gene amplification techniques in which cloning vectors are used. The techniques listed above would be readily performed by those skilled in the art, according to Molecular cloning 2nd Edt. Cold Spring Harbor Laboratory Press (1989), etc. For purification of the resulting PCR products, any known method may be used.

Amyloid β protein (Aβ) is a polypeptide composed of 40 to 43 amino acids, which is cleaved from its precursor protein (APP: amyloid β protein precursor) by the action of β- and γ-secretases.

To present antigenicity against Aβ, such a polypeptide comprises 15 or more contiguous amino acids, preferably 20 or more contiguous amino acids derived from the native Aβ amino acid sequence. A polypeptide comprising the native full-length Aβ may also be used.

The nucleotide sequences of these DNAs are available from a certain database. For example, Genbank Accession No. NC_000021.7 may be used for human Aβ, while Accession No. NC_000082.5 may be used for mouse Aβ.

In a preferred embodiment of the present disclosure, the above DNAs may be used as templates for PCR with primers specific for these genes to prepare each DNA region with each fragment length. In the present disclosure, it is possible to use a polypeptide of a region covering 43 amino acids cleaved by the action of γ-secretase (referred to as "Aβ1-43"), a region covering N-terminal Aβ amino acids 1 to 20 (referred to as "Aβ1-20"), a region covering N-terminal amino acids 1 to 40 (referred to as "Aβ1-40"), or a region covering N-terminal amino acids 1 to 42 (referred to as "Aβ1-42"). The amino acid sequences of human Aβ1-43, Aβ1-20, Aβ1-40 and Aβ1-42 are shown in SEQ ID NOs: 2, 4, 6 and 8, respectively, while those of mouse Aβ1-43, Aβ1-20, Aβ1-40 and Aβ1-42 are shown in SEQ ID NOs: 10, 12, 14 and 16, respectively.

The nucleotide sequences of DNAs encoding human Aβ1-43, Aβ1-20, Aβ1-40 and Aβ1-42 are shown in SEQ ID NOs: 1, 3, 5 and 7, respectively, while the nucleotide sequences of DNAs encoding mouse Aβ1-43, Aβ1-20, Aβ1-40 and Aβ1-42 are shown in SEQ ID NOs: 9, 11, 13 and 15, respectively.

DNAs encoding human or mouse Aβ1-20, Aβ1-40 and Aβ1-42 may be prepared by PCR or other techniques from DNA encoding human or mouse Aβ1-43.

In addition to the above DNAs encoding human or mouse Aβ1-43, Aβ1-20, Aβ1-40 and Aβ1-42, the following DNAs may also be used in the present invention.

DNA which is hybridizable under stringent conditions with DNA consisting of a nucleotide sequence complementary to the nucleotide sequence shown in SEQ ID NO: 1 and which encodes a protein having human Aβ activity.

DNA which is hybridizable under stringent conditions with DNA consisting of a nucleotide sequence complementary to the nucleotide sequence shown in SEQ ID NO: 3 and which encodes a protein having human Aβ activity.

DNA which is hybridizable under stringent conditions with DNA consisting of a nucleotide sequence complementary to the nucleotide sequence shown in SEQ ID NO: 5 and which encodes a protein having human Aβ activity.

DNA which is hybridizable under stringent conditions with DNA consisting of a nucleotide sequence complementary to the nucleotide sequence shown in SEQ ID NO: 7 and which encodes a protein having human Aβ activity.

DNA which is hybridizable under stringent conditions with DNA consisting of a nucleotide sequence complementary to the nucleotide sequence shown in SEQ ID NO: 9 and which encodes a protein having mouse Aβ activity.

DNA which is hybridizable under stringent conditions with DNA consisting of a nucleotide sequence complementary to the nucleotide sequence shown in SEQ ID NO: 11 and which encodes a protein having mouse Aβ activity.

DNA which is hybridizable under stringent conditions with DNA consisting of a nucleotide sequence complementary to the nucleotide sequence shown in SEQ ID NO: 13 and which encodes a protein having mouse Aβ activity.

DNA which is hybridizable under stringent conditions with DNA consisting of a nucleotide sequence complementary to the nucleotide sequence shown in SEQ ID NO: 15 and which encodes a protein having mouse Aβ activity.

In the context of the present invention, the term "Aβ activity" is intended to mean the ability to produce, accumulate and/or aggregate Aβ in the brain of a subject (e.g., human, mouse) to thereby form Aβ deposition (senile plaques). Aβ activity can be measured by immunological procedures such as immunohistological staining, ELISA and the like. For example, in the case of immunohistological staining, a protein to be evaluated may be expressed in the brain of a subject animal (e.g., mouse), and sections of the expressed tissue may be immunostained with anti-Aβ antibody to detect Aβ production, accumulation, aggregation and/or deposition for activity assay.

Human Aβ1-43, human Aβ1-20, human Aβ1-40, human Aβ1-42, mouse Aβ1-43, mouse Aβ1-20, mouse Aβ1-40 and mouse Aβ1-42 have their own Aβ activity. Thus, for example, in the case of a protein expressed from DNA which is hybridizable under stringent conditions with DNA consisting of a nucleotide sequence complementary to the nucleotide sequence shown in SEQ ID NO: 1 and which encodes a protein having human Aβ activity, it is sufficient for this protein to have Aβ activity equal to that of human Aβ1-43. The same also applies to proteins expressed from DNAs which are hybridizable under stringent conditions with DNAs consisting of nucleotide sequences complementary to the nucleotide sequences shown in the other SEQ ID NOs and which encode proteins having Aβ activity.

Stringent conditions in the above hybridization include, for example, conditions of 0.1 x SSC to 10 x SSC, 0.1% to 1.0% SDS and 20°C to 80°C, more specifically prehybridization at 37°C to 56°C for 30 minutes or longer and the subsequent one to three washings in 0.1 x SSC, 0.1% SDS at room temperature for 10 to 20 minutes. For detailed procedures of hybridization, reference may be made to "Molecular Cloning, A Laboratory Manual 2nd ed." (Cold Spring Harbor Press (1989)), etc.

It is also possible to use DNA which shares a homology of 50% or more, 60% or more, 70% or more, 80% or more, 90% or more, 95% or more, 98% or more, or 99% or more with the nucleotide sequence shown in SEQ ID NO: 1, 3, 5 or 7 and which encodes a protein functionally equivalent to the structural protein of human Aβ1-43, Aβ1-20, Aβ1-40 or Aβ1-42.

It is further possible to use DNA which shares a homology of 50% or more, 60% or more, 70% or more, 80% or more, 90% or more, 95% or more, 98% or more, or 99% or more with the nucleotide sequence shown in SEQ ID NO: 9, 11, 13 or 15 and which encodes a protein functionally equivalent to the structural protein of mouse Aβ1-43, Aβ1-20, Aβ1-40 or Aβ1-42.

The term "functionally equivalent" is intended to mean having the function of causing Aβ deposition (senile plaques) in tissues, etc.

In the present invention, DNA encoding Aβ consists of repeats of the DNA encoding Aβ. A vector comprising repeats of Aβ-encoding DNA allows multiple Aβ molecules to be expressed simultaneously. Moreover, Aβ molecules released into the extracellular environment would form an oligomer. This oligomer stimulates the immune system to induce anti-Aβ oligomer antibody. As a result, it can be expected to eliminate Aβ oligomers whose neurotoxicity is stronger than that of Aβ monomers.

There is no limitation on the repeated number of Aβ-encoding DNAs as long as the folded structure of Aβ is formed to give higher antigenicity than that of Aβ monomers. The number of repeats preferably ranges from 2 to 4, more preferably from 3 to 4.

In the context of the present invention, the term "Th2" is intended to mean a subgroup of CD4⁺ T cells (commonly called helper T cells), and refers to cells that are induced to differentiate from T cells not experiencing any contact with antigen proteins (i.e., naive T cells) upon stimulation with cytokines such as IL-4 or IL-13. Th2 is involved in regulation of humoral immunity, and cytokines produced from Th2 promote antibody production. Examples of Th2 cytokines include IL-4 (interleukin-4), IL-5, IL-6, IL-10, IL-13, M-CSF (macrophage-colony-stimulating factor) and so on. The Th2 cytokine of interest for the instant invention is IL-4.

Genbank Accession Nos. NM_000589.2 and NM_021283.1 may be used for human IL-4 and mouse IL-4, respectively. Likewise, Genbank Accession Nos. NM_000757.4 and NM_007778.3 may be used for human M-CSF and mouse M-CSF, respectively, as reference.

SEQ ID NOs of the nucleotide sequences of the above DNAs encoding IL-4 and M-CSF are shown below.
Human IL-4: SEQ ID NO: 17
Mouse IL-4: SEQ ID NO: 18
Human M-CSF: SEQ ID NO: 19
Mouse M-CSF: SEQ ID NO: 20

In addition to the above DNAs encoding the Th2 cytokines IL-4, the following DNAs may also be used in the present invention.

DNA which is hybridizable under stringent conditions with DNA consisting of a nucleotide sequence complementary to the nucleotide sequence shown in SEQ ID NO: 17 and which encodes a protein having human IL-4 activity.

DNA which is hybridizable under stringent conditions with DNA consisting of a nucleotide sequence complementary to the nucleotide sequence shown in SEQ ID NO: 18 and which encodes a protein having mouse IL-4 activity.

The term "IL-4 activity" is intended to mean the ability to promote Th2 cell differentiation, immunoglobulin class switch, and antibody production in B cells. IL-4 activity can be measured, for example, by immunological procedures such as ELISA, EIA and the like.

The term "M-CSF activity" is intended to mean the ability to promote differentiation and proliferation of monocytes, macrophages and other cells. M-CSF activity can be measured, for example, by immunological procedures such as ELISA, EIA and the like.

Moreover, it is also possible to use DNA which shares a homology of 50% or more, 60% or more, 70% or more, 80% or more, 90% or more, 95% or more, 98% or more, or 99% or more with the nucleotide sequence shown in SEQ ID NO: 17 and which encodes a protein functionally equivalent to the structural protein of human IL-4.

Further, it is also possible to use DNA which shares a homology of 50% or more, 60% or more, 70% or more, 80% or more, 90% or more, 95% or more, 98% or more, or 99% or more with the nucleotide sequence shown in SEQ ID NO: 18 and which encodes a protein functionally equivalent to the structural protein of mouse IL-4.

The immunoglobulin Fc (IgFc) sequence to be used in the present invention refers to a C-terminal fragment of the H chain, which is among the fragments obtained by papain digestion of immunoglobulin molecules.

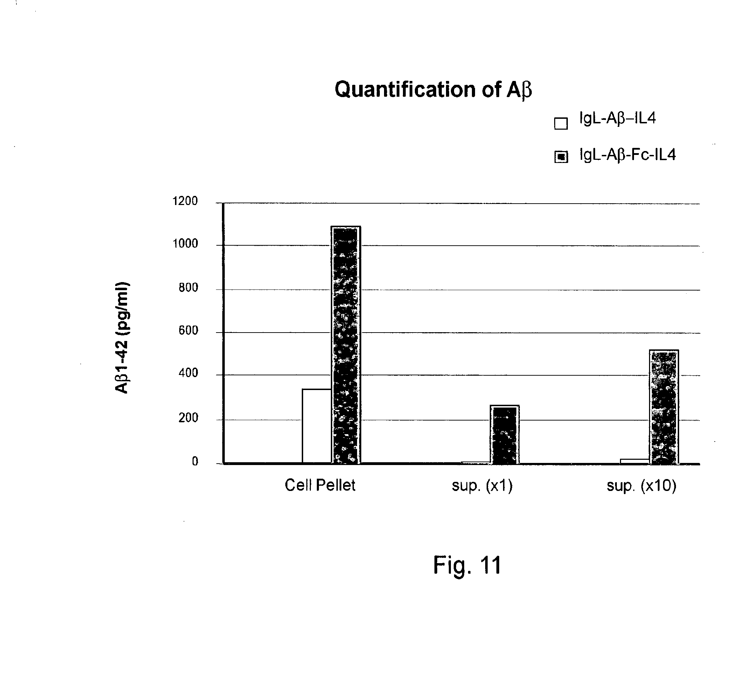

In the present invention, a vector comprising DNA encoding an IgFc sequence allows addition of the IgFc sequence to Aβ to thereby promote intracellular transcription and translation of Aβ. Moreover, such a vector also enhances release of the expressed Aβ into the extracellular environment, so that the immune system can be stimulated more strongly (see Figure 11).

Examples of IgFc sequences to be used in the present invention include human and mouse IgFc sequences. With respect to the nucleotide sequences of DNAs encoding human and mouse IgFc sequences, Genbank Accession Nos. BC014258 and XM_484178.3 may be used for human and mouse IgFc sequences, respectively.

SEQ ID NOs of the nucleotide sequences of DNAs encoding human and mouse IgFc sequences to be used in the present invention are shown below.
Human IgFc sequence: SEQ ID NO: 32
Mouse IgFc sequence: SEQ ID NO: 33

The nucleotide sequences shown in SEQ ID NOs: 32 and 33 each have a mutation to replace cysteine residues contained in the original IgFc sequence with serine residues. This is intended to avoid the formation of disulfide linkages.

Moreover, the following DNAs may also be used as DNAs encoding human and mouse IgFc sequences to be used in the present invention.

DNA which is hybridizable under stringent conditions with DNA consisting of a nucleotide sequence complementary to the nucleotide sequence shown in SEQ ID NO: 32 and which encodes a protein having human IgFc activity.

DNA which is hybridizable under stringent conditions with DNA consisting of a nucleotide sequence complementary to the nucleotide sequence shown in SEQ ID NO: 33 and which encodes a protein having mouse IgFc activity.

In the context of the present invention, the term "IgFc activity" is intended to mean the ability to promote intracellular production and extracellular release of Aβ protein. For example, the IgFc activity of a certain protein may be measured as follows: a fusion protein between this protein and Aβ protein is expressed in cultured cells, and an increase in the amount of Aβ protein present in the cultured cells or in the culture supernatant is determined. Quantification of Aβ protein may be accomplished by using immunological procedures such as ELISA, EIA and the like.

Moreover, in the present invention, it is also possible to use DNA which shares a homology of 50% or more, 60% or more, 70% or more, 80% or more, 90% or more, 95% or more, 98% or more, or 99% or more with the nucleotide sequence shown in SEQ ID NO: 32 and which encodes a protein having human IgFc activity.

Further, it is also possible to use DNA which shares a homology of 50% or more, 60% or more, 70% or more, 80% or more, 90% or more, 95% or more, 98% or more, or 99% or more with the nucleotide sequence shown in SEQ ID NO: 33 and which encodes a protein having mouse IgFc activity.

IgFc activity is as defined above.

The above DNAs may be obtained by chemical synthesis or may be obtained from cDNA and genomic libraries by known hybridization techniques (e.g., colony hybridization, plaque hybridization, Southern blotting) using DNA consisting of the nucleotide sequence shown in SEQ ID NO: 1, 3, 5, 7, 9, 11, 13, 15, 17, 18, 19, 20, 32 or 33 or a fragment thereof as a probe.

To construct recombinant vectors, standard genetic engineering procedures may be used.

For example, samples of DNA encoding Aβ of interest, DNA encoding a Th2 cytokine and DNA encoding an IgFc sequence are prepared by PCR or other techniques. PCR may be accomplished in a standard manner by using Taq polymerase or other DNA polymerases. The amplified fragments of interest are digested with restriction enzymes and then inserted into restriction enzyme sites or a multicloning site in a plasmid vector such as pBluescript^{™} (Stratagene). The resulting PCR products are confirmed for their nucleotide sequences with a sequencer to select a plasmid containing the proper sequence. It is preferred that such a DNA sample can be confirmed as an electrophoretically single plasmid.

As a promoter contained in the recombinant vector of the present invention, actin promoter, EF1 promoter, CMV promoter, CAG promoter or the like may be used. These promoters may each be ligated to an appropriate plasmid.

"Stringent conditions" are as defined above. The phrase "having promoter activity" is intended to mean having the ability to transcribe a gene encoding a structural protein or a non-structural protein.

In the vector of the present invention, the above Aβ and Th2 cytokine, and the IgFc sequence are contained in operable form to ensure their expression. Namely, the transgenes (DNAs) are inserted into the vector in a mode which allows expression of the transgenes under the control of appropriate regulatory elements. The DNAs encoding Aβ, Th2 cytokine and IgFc sequence may be separately inserted into different sites in the same vector or may be inserted in tandem. In this context, regulatory elements refer to, for example, promoters, enhancers, transcription terminators and so on.

The vector of the present invention may carry an additional foreign gene at a position different from the region(s) into which the DNAs encoding Aβ, Th2 cytokine and IgFc sequence are inserted. Such a foreign gene may be, for example, but is not limited to, a marker gene for vector monitoring or a regulatory gene for the immune system, such as cytokines and hormones.

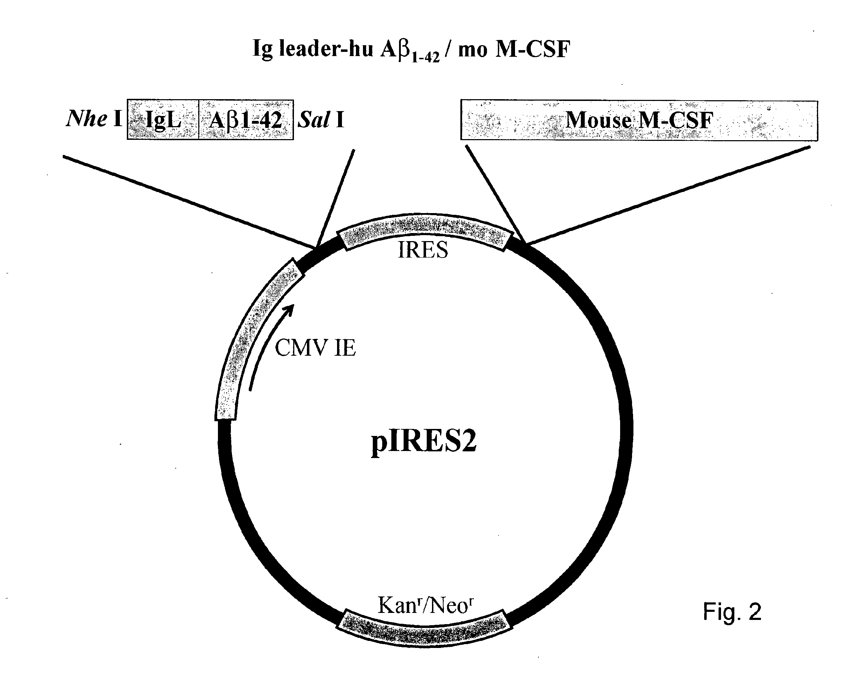

A reference vector may be exemplified by pTARGET in which the Aβ1-42 gene and the IL-4 gene are inserted downstream of the CMV promoter (Figure 1), as well as pIRES2 in which the Aβ1-42 gene and the M-CSF gene are inserted downstream of the CMV promoter (Figure 2).

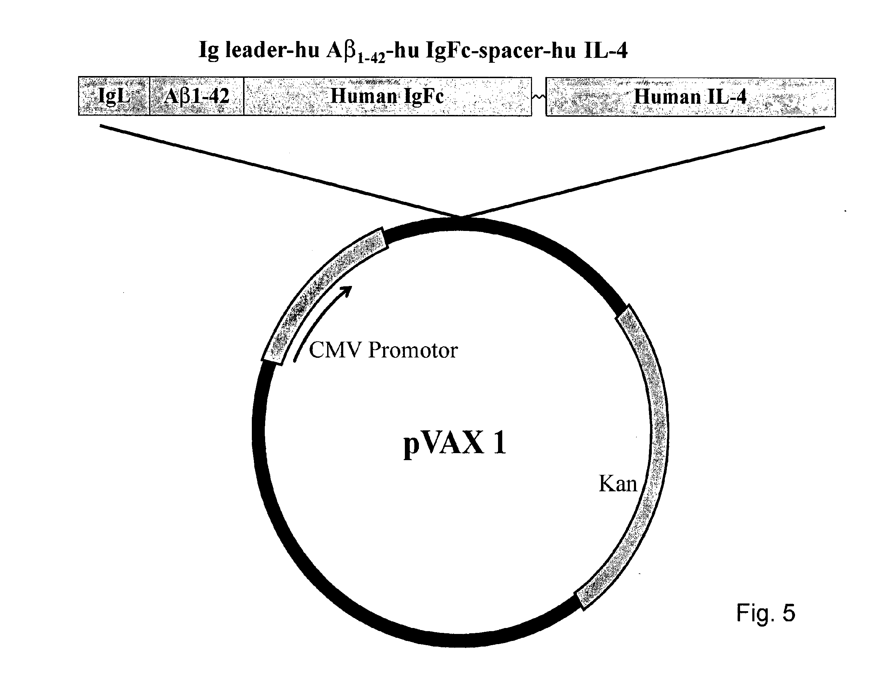

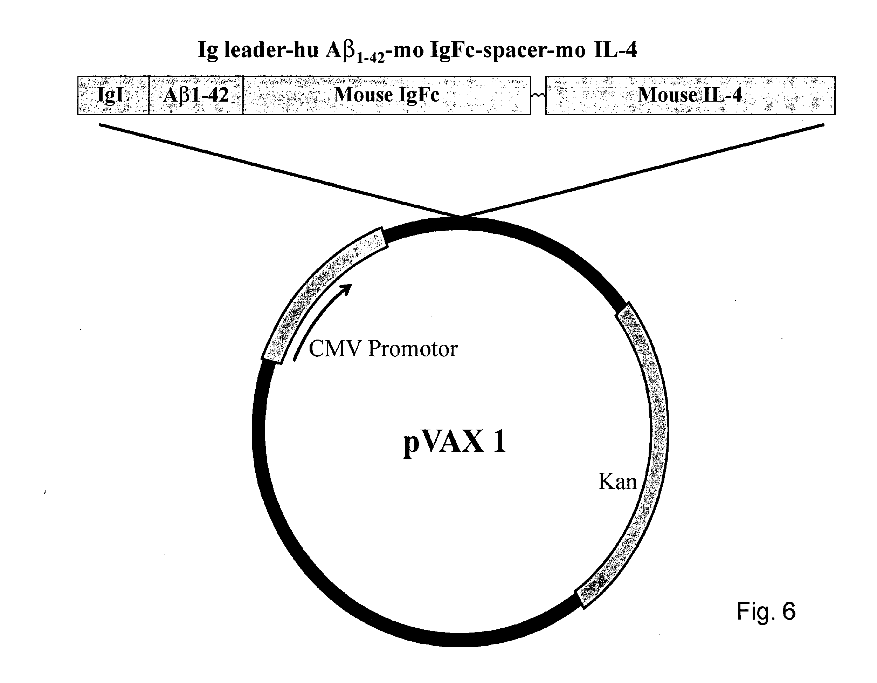

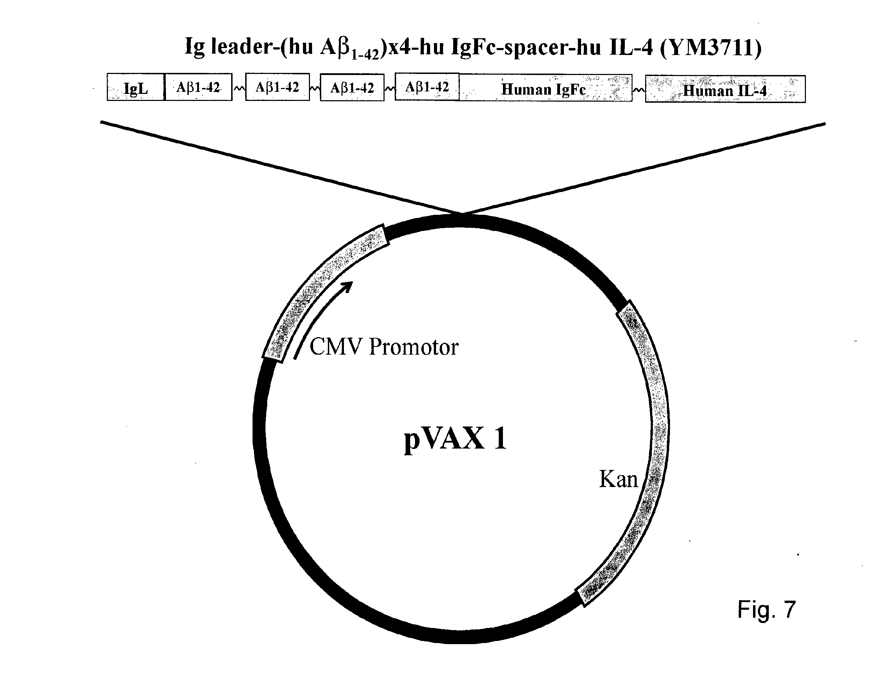

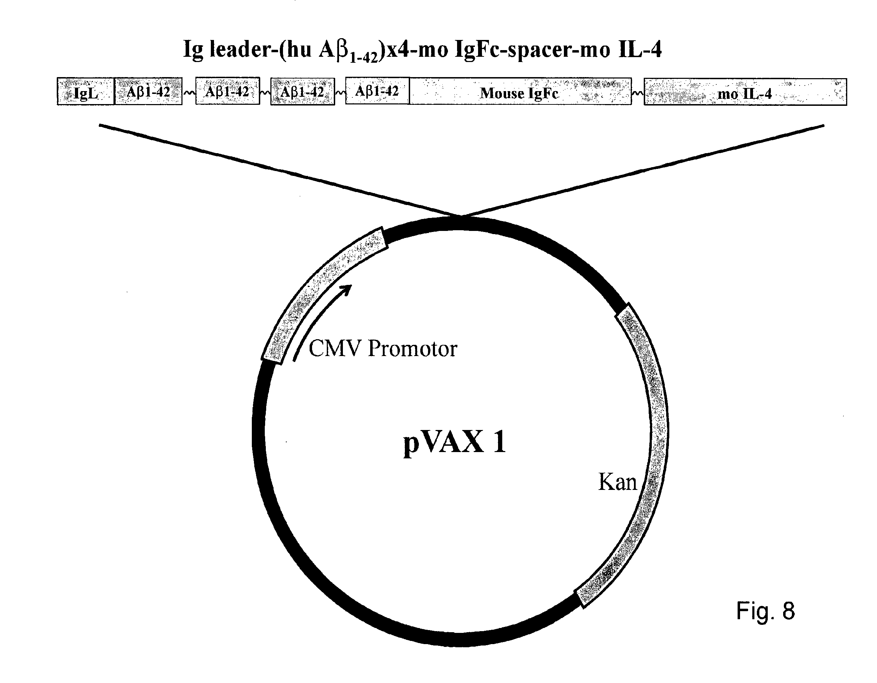

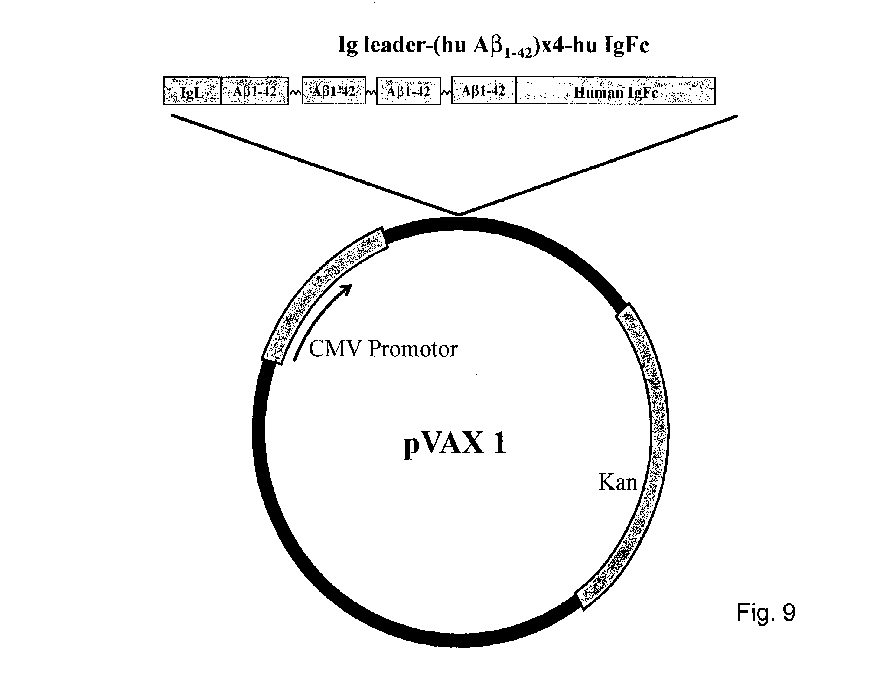

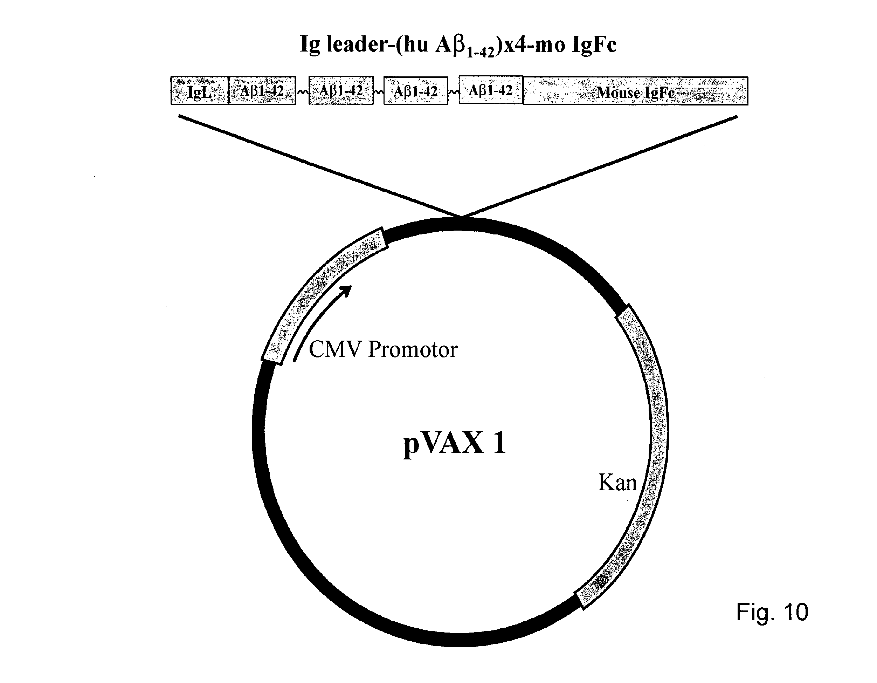

Another reference vector may also be exemplified by pVAX1 in which the Aβ1-42 gene, the IgFc gene and the IL-4 gene are inserted downstream of the CMV promoter (Figures 5 and 6). A vector of the present invention may be exemplified by pVAX1 in which repeats of the Aβ1-42 gene (DNA), the IgFc gene and the IL-4 gene are inserted downstream of the CMV promoter (Figures 7 and 8). A further reference vector is pVAX1 in which repeats of the Aβ1-42 gene and the IgFc gene are inserted downstream of the CMV promoter (Figures 9 and 10).

The reference vector pTARGET, in which the Aβ1-42 gene and the IL-4 gene are inserted downstream of the CMV promoter, preferably comprises an Ig leader (IgL) sequence upstream of the Aβ1-42 gene and a spacer sequence between the Aβ1-42 gene and the IL-4 gene. More preferably, this pTARGET comprises the CMV promoter, the Ig leader sequence, the Aβ1-42 gene, the spacer sequence and the IL-4 gene in this order (Figure 1).

Likewise, the reference vector pIRES2, in which the Aβ1-42 gene and the M-CSF gene are inserted downstream of the CMV promoter, preferably comprises an Ig leader sequence upstream of the Aβ1-42 gene and an IRES sequence between the Aβ1-42 gene and the M-CSF gene. More preferably, this pIRES2 comprises the CMV promoter, the Ig leader sequence, the Aβ1-42 gene, the IRES sequence and the M-CSF gene in this order (Figure 2).

The reference vector pVAX1, in which the Aβ1-42 gene, the IgFc gene and the IL-4 gene are inserted downstream of the CMV promoter, preferably comprises an Ig leader sequence between the CMV promoter and the Aβ1-42 gene and a spacer sequence between the IgFc gene and the IL-4 gene. More preferably, this pVAX1 comprises the CMV promoter, the Ig leader sequence, the Aβ1-42 gene, the IgFc gene, the spacer sequence and the IL-4 gene in this order (Figures 5 and 6).

pVAX1 of the present invention, in which repeats of the Aβ1-42 gene, the IgFc gene and the IL-4 gene are inserted downstream of the CMV promoter, preferably comprises an Ig leader sequence between the CMV promoter and repeats of the Aβ1-42 gene, as well as spacer sequences between the individual Aβ1-42 genes and between the IgFc gene and the IL-4 gene. More preferably, this pVAX1 comprises the CMV promoter, the Ig leader sequence, repeats of the Aβ1-42 gene (containing spacer sequences between the individual Aβ1-42 genes), the IgFc gene, the spacer sequence and the IL-4 gene in this order (Figures 7 and 8).

The reference vector pVAX1, in which repeats of the Aβ1-42 gene and the IgFc gene are inserted downstream of the CMV promoter, preferably comprises an Ig leader sequence between the CMV promoter and repeats of the Aβ1-42 gene, as well as spacer sequences between the individual Aβ1-42 genes. More preferably, this pVAX1 comprises the CMV promoter, the Ig leader sequence, repeats of the Aβ1-42 gene (containing spacer sequences between the individual Aβ1-42 genes) and the IgFc gene in this order (Figures 9 and 10).

The above vectors each have genes of mouse or human origin. The vectors having genes of mouse origin can be used in preclinical trials or reagents, while the vectors having genes of human origin can be used in pharmaceutical compositions or reagents.

Namely, the present invention provides a pharmaceutical composition (DNA vaccine) for prevention or treatment of Alzheimer's disease, which comprises the above vector. Also disclosed is a pharmaceutical composition (DNA vaccine) for elimination of brain Aβ, which comprises the above vector. In the context of the present application, the term "elimination" or "eliminating" is intended to mean reducing the amount of Aβ present in the brain, which encompasses reducing the amount of brain Aβ accumulated, aggregated or deposited in the brain. Moreover, the pharmaceutical composition, which comprises the above vector, may also be used as a DNA vaccine for suppressing an increase in brain Aβ levels.

In yet another embodiment, the present invention provides an inducer of anti-Aβ antibody, which comprises the above vector. In still yet another embodiment, the present invention provides an inducer of anti-pEAβ3-42 antibody, anti-ABri antibody or anti-ADan antibody, which comprises the above vector.

pEAβ3-42 is a molecule obtained from Aβ1-42 by N-terminal truncation with gulutaminyl cyclase (QC) and post-translational modification (pyrrole modification). pEAβ3-42 is highly neurotoxic and this molecule itself has a high tendency to aggregate. It also has the ability to enhance the aggregation propensity of unmodified Aβ, and is one of the major factors responsible for lesion development in Alzheimer's disease.

ABri is a causative molecule for familial British dementia, while ADan is a causative molecule for familial Danish dementia. ABri and ADan are molecules which are cleaved as long molecules from their precursor proteins due to a gene mutation in the stop codon region of each precursor protein. ABri and ADan have high amyloid aggregation propensity (amyloid is a generic name for events in which small molecules are aggregated and deposited) and are key molecules in disease progression. Thus, they appear to also play some role in the development ofAlzheimer lesions.

In a case where the vector of the present invention is used as a DNA vaccine or as an inducer of anti-Aβ antibody, anti-pEAβ3-42 antibody, anti-ABri antibody or anti-ADan antibody (hereinafter also referred to as "inducers of anti-Aβ antibody and others"), gene transfer may be accomplished either by direct administration in which the vector is directly injected into a target site in the body or by indirect administration in which the vector is infected into patient's own cells or other cells for gene transfer, and the infected cells are then injected into a target site. For direct injection of the vector, intramuscular injection or the like may be used.

Alternatively, the vector of the present invention may also be administered by being introduced into phospholipid vesicles such as liposomes. The vesicles holding the vector of the present invention are introduced by lipofection into certain specific cells. Then, the resulting cells are administered systemically, for example, by the intravenous or intraarterial route. They may be administered locally to the brain, etc.

Examples of lipids used to form liposome structures include phospholipids, cholesterols and nitrogen-containing lipids. Commonly preferred are phospholipids, including natural phospholipids such as phosphatidylcholine, phosphatidylserine, phosphatidylglycerol, phosphatidylinositol, phosphatidylethanolamine, phosphatidic acid, cardiolipin, sphingomyelin, egg yolk lecithin, soybean lecithin, and lysolecithin, as well as hydrogenated products thereof obtained in a standard manner. It is also possible to use synthetic phospholipids such as dicetyl phosphate, distearoylphosphatidylcholine, dipalmitoylphosphatidylcholine, dipalmitoylphosphatidylethanolamine, dipalmitoylphosphatidylserine, eleostearoylphosphatidylcholine, eleostearoylphosphatidylethanolamine and so on.

The preparation of liposomes is not limited in any way as long as the resulting liposomes hold DNAs. Liposomes may be prepared in a conventional manner, for example, by reversed-phase evaporation, ether injection, surfactant-based techniques, etc.

Lipids including these phospholipids may be used either alone or in combination. In this case, it is also possible to increase the binding rate of electrically negative DNA when using a lipid whose molecule contains an atomic group having a cationic group (e.g., ethanolamine or choline). In addition to these major phospholipids used for liposome formation, it is also possible to use other additives such as cholesterols, stearyl amine, α-tocopherol and the like, which are generally known as liposome-forming additives. The liposomes thus obtained may further comprise a membrane fusion promoter (e.g., polyethylene glycol) in order to enhance their uptake into cells in the affected area or target tissue.

The vaccine of the present invention or the inducers of anti-Aβ antibody and others according to the present invention may be formulated in a routine manner and may comprise pharmaceutically acceptable carriers. Such carriers may be additives and include water, pharmaceutically acceptable organic solvents, collagen, polyvinyl alcohol, polyvinylpyrrolidone, carboxyvinyl polymers, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerine, glycerine, propylene glycol, petrolatum, paraffin, stearyl alcohol, stearic acid, human serum albumin, mannitol, sorbitol, lactose, and surfactants acceptable as pharmaceutical additives, etc.

The above additives may be selected alone or in combination from among those listed above, depending on the dosage form of each therapeutic agent of the present invention. For example, for use as injectable formulations, the purified vector may be dissolved in a solvent (e.g., physiological saline, buffer, glucose solution) and then supplemented with Tween 80, Tween 20, gelatin, human serum albumin or the like. Alternatively, the ingredients may be lyophilized for use as dosage forms that are reconstituted before use. Examples of excipients for lyophilization include sugars such as mannitol, glucose, lactose, sucrose, mannitol and sorbitol; starches such as those derived from corn, wheat, rice, potato and other plants; celluloses such as methylcellulose, hydroxypropylmethylcellulose and carboxymethylcellulose sodium; gums such as gum arabic and gum tragacanth; as well as gelatin, collagen and so on.

Targets to be administered with the DNA vaccine of the present invention or with the inducers of anti-Aβ antibody and others according to the present invention include, for example, humans and all other mammals such as non-human primates (e.g., monkeys), rodents (e.g., mice and rats), rabbits, goats, sheep, pigs, cattle and dogs, with humans being more preferred. Animals serving as targets to be administered may be, for example, those suffering from Alzheimer's disease, those suspected to have Alzheimer's disease, those showing enhanced Aβ deposition, those showing enhanced tau deposition, or those showing neuronal loss.

The dosage of the DNA vaccine of the present invention or the inducers of anti-Aβ antibody and others according to the present invention will vary depending on the age, sex and symptoms of a patient, the route of administration, the frequency of administration, and the intended dosage form. The mode of administration is selected as appropriate for the age and symptoms of a patient. The effective dosage of the DNA vaccine is an amount of the vaccine required to reduce the signs or condition of the disease. The therapeutic effect and toxicity of such a DNA vaccine may be determined by standard pharmaceutical procedures in cell culture or in laboratory animals, for example, by ED50 (therapeutically effective dose in 50% of the population) or LD50 (lethal dose for 50% of the population) assay. Likewise, the effective dosage of the inducer of anti-Aβ antibody is an amount of the inducer required to induce detectable levels of anti-Aβ antibody in a biological sample (including blood, cell, tissue, etc.) collected from a patient. Detection of anti-Aβ antibody may be accomplished by immunological procedures such as ELISA, immunostaining and the like. Those skilled in the art would be able to determine an appropriate dosage for the DNA vaccine or the inducer of anti-Aβ antibody.

The route of administration may be selected as appropriate and examples include, but are not limited to, percutaneous, intranasal, transbronchial, intramuscular, intraperitoneal, intravenous and subcutaneous routes. Particularly preferred are intramuscular administration, subcutaneous administration and so on. Inoculation may be made at a single site or at multiple sites.

The dose ratio between therapeutic and toxic effects is a therapeutic index and can be expressed as ED50/LD50. In humans, the single dosage of the vaccine of the present invention or the inducer of anti-Aβ antibody according to the present invention is about 1 µg to 1000 µg, preferably about 10 to 500 µg, more preferably about 50 to 250 µg. The frequency of administration may be once or more as long as side effects are within a clinically acceptable range.

In the previous development of vaccines for Alzheimer's disease, studies have been conducted focusing on anti-Aβ antibody and Th2 activity. Thus, it is desirable to measure, in advance, the antibody titer or cellular immune activity as a vaccine.

For example, the cellular immune activity may be evaluated by isolating and culturing lymphocytes from the body and measuring their ³H-thymidine uptake.

Likewise, the Th2 activity may be evaluated by isolating plasma from peripheral blood and measuring its antibody titer by ELISA.

Once the DNA vaccine of the present invention is administered to a target animal, immune responses against Aβ are induced. Namely, since the above amino acid sequence of Aβ1-43, Aβ1-20, Aβ1-40 or Aβ1-42 contains an epitope, antibody production is induced when the vaccine of the present invention is administered.

Immune responses against Aβ may be detected by measuring the level of anti-Aβ antibody produced. The level of the antibody produced may be measured by standard immunological procedures such as ELISA (enzyme-linked immunosorbent assay). Likewise, the therapeutic effect of the vaccine may be confirmed, for example, as a reduction in the amount of Aβ in brain tissues or as a reduction in Aβ deposition (senile plaques). The amount of Aβ in brain tissues or the state of Aβ deposition may be observed by immunohistochemical or other techniques.

The present invention will be further described in more detail by way of the following illustrative examples, which are not intended to limit the scope of the invention.

### [Example 1]

### 1. Construction of plasmid vector pTarget/Ig-leader-Aβ1-42-IgFc

### (1) Amplification and cloning of IgL, Aβ and Fc genes

To clone the sequences of immunoglobulin κ leader (hereinafter referred to as IgL) and immunoglobulin Fc (hereinafter referred to as Fc or IgFc), human peripheral blood mRNA was used as a material to synthesize cDNAs with ReverTra Ace-α-(TOYOBO, Tokyo, Japan). Primers containing the 5'- or 3'-terminal end of each sequence and having an appropriate restriction enzyme site (IgL: BamH I or Xho I; Fc: Kpn I or Not I) were designed and used to amplify human IgL and Fc sequences with KOD-plus- (Toyobo, Tokyo, Japan). Although the original sequence of human Fc contains three codons each encoding a cysteine residue near the 5'-terminal end, these codons were each modified to encode a serine residue (TGT → TCT or TGC → TCC) during primer design so as to avoid S-S linking, and the primers thus designed were used to obtain amplification products.

The sequence of amyloid β1-42 (hereinafter referred to as Aβ) was prepared by oligonucleotide synthesis, provided that two oligonucleotides were first synthesized, which contained the 5'- or 3'-terminal end of the Aβ sequence and were partially complementary to each other (24 bp in the middle of the Aβ sequence), because a sequence covering the full length (126 bp) was difficult to synthesize. An appropriate restriction enzyme site (Xho I, Kpn I) was added to each end. After these oligonucleotides were annealed, the entire duplex was prepared by polymerase reaction.

Each product obtained above was inserted into a cloning vector (IgL and Fc: Zero Blunt TOPO PCR Kit for Sequencing, Invitrogen, Tokyo, Japan; Aβ: AdvanTAge PCR Cloning Kit, TAKARA, Tokyo, Japan), followed by sequencing to select a clone having the desired sequence.

### (2) Insertion of IgL, Aβ and Fc sequences into expression vector

DNAs of the IgL, Aβ and Fc sequences were excised from the cloning vectors by cleavage at the restriction enzyme sites previously added to each sequence, and then purified from an agarose gel (MinElute Gel Extraction Kit, Qiagen, Tokyo, Japan). Without using the TA cloning site, the pTarget vector was cleaved at two restriction enzyme sites Xho I and Kpn I located upstream and downstream of the TA cloning site to give the same overhangs as those of the Aβ sequence, and then purified from an agarose gel (MinElute Gel Extraction Kit).

First, the pTarget vector and the Aβ sequence were ligated together using a Ligation high kit (Toyobo, Tokyo, Japan) to construct pTarget/Aβ. Subsequently, the region immediately upstream of the Aβ sequence was treated with restriction enzymes BamH I and Xho I for integration of the IgL sequence to thereby construct pTarget/IgL-Aβ. Further, the Kpn I and Not I sites immediately downstream of the Aβ sequence were cleaved for ligation of the Fc sequence to complete pTarget/IgL-Aβ-Fc.

### 2. Preparation of pTarget/Ig leader-Amyloid β 1-42-IL-4 (IgL-Aβ-IL-4) vaccine

### (1) Amplification of IL-4 sequence

Ig leader-Amyloid β 1-42-IL-4 (IgL-Aβ-IL-4) (Figure 1) was prepared using pTarget/Ig leader-Amyloid β 1-42-IgFc (IgL-Aβ-Fc) described above. First, human peripheral blood mRNA and mouse spleen mRNA were used to synthesize cDNAs with ReverTra Ace-α- (TOYOBO, Tokyo, Japan). Primers containing the 5'- or 3'-terminal end of human or mouse IL-4 mRNA (coding region) and having an appropriate restriction enzyme site (Kpn I or Not I) were designed and used to amplify the full-length human and mouse IL-4 sequences with KOD-plus- (Toyobo, Tokyo, Japan). PCR products were each purified from an agarose gel (MinElute Gel Extraction Kit, Qiagen, Tokyo, Japan) and inserted into a cloning vector (Zero Blunt TOPO PCR Kit for Sequencing, Invitrogen, Tokyo, Japan), followed by sequencing to select a clone having the desired sequence. For human IL-4, the sequences of both isotypes 1 (hIL-4) and 2 (hIL-4δ2), which were splice variants, were amplified. However, the latter is a receptor antagonist of IL-4 (Alms WJ et al, Mol Immunol 1996, 33:361-370; Fletcher HA et al, Immunology 2004, 112:669-673; Plante S. et al, J Allergy Clin Immunol 2006, 117:1321-1327), and hence isotype 1 was selected.

### (2) Recombination to replace Fc sequence with IL-4 sequence

The IL-4 sequence was excised from the cloning vector by cleavage at the previously added restriction enzyme sites (Kpn I, Not I), while the Fc sequence was excised from pTarget/IgL-Aβ-Fc with the same restriction enzyme pair. The hIL-4 or mIL-4 sequence and pTarget/IgL-Aβ were each purified from an agarose gel (MinElute Gel Extraction Kit) and then ligated together overnight at 16°C using a Ligation high kit (Toyobo, Tokyo, Japan), followed by transformation into TOP10F' (Invitrogen, Tokyo, Japan) and blue-white selection to select a clone carrying the desired sequence (i.e., an insert of the correct size) by restriction enzyme cleavage. The nucleotide sequence of the insert in pTarget/IgL-Aβ1-42-hIL-4 thus obtained is shown in SEQ ID NO: 21, while the nucleotide sequence of the insert in pTarget/IgL-Aβ1-42-mIL-4 thus obtained is shown in SEQ ID NO: 22.

### 3. Preparation of pIRES2/Ig leader-Amyloid β 1-42-M-CSF (IgL-Aβ-M-CSF) vaccine

Since M-CSF is a protein that exerts its functions upon homodimer formation, Aβ1-42 and M-CSF were expressed as separate proteins with an IRES system.

### (1) Amplification of M-CSF sequence and Ig leader (IgL) sequence

Mouse spleen mRNA was used to synthesize cDNA with ReverTra Ace-α-(TOYOBO, Tokyo, Japan). Primers containing the 5'- or 3'-terminal end of mouse M-CSF (moM-CSF) mRNA (coding region) and having any appropriate restriction enzyme site (BamH I or Not I) were designed and used to amplify the full-length mouse M-CSF sequence with KOD-plus- (Toyobo, Tokyo, Japan). The Ig leader (IgL) sequence was amplified with primers having necessary restriction enzyme sites (Nhe I, Xho I) by using pTarget/Ig leader-Amyloid β 1-42-IgFc (pTarget/IgL-Aβ-Fc) as a template. The PCR products were each purified from an agarose gel (MinElute Gel Extraction Kit, Qiagen, Tokyo, Japan) and inserted into a cloning vector (Zero Blunt TOPO PCR Kit for Sequencing, Invitrogen, Tokyo, Japan), followed by sequencing to select a clone having the desired sequence.

### (2) Recombination to replace APL sequence with IgL sequence

Based on the previously prepared vector pIRES2-EGFP/Alkaline phosphatase leader-Amyloid β 1-42 (pIRES2-EGFP/APL-Aβ) (pIRES2-EGFP vector: Takara-Chlontech, Tokyo, Japan), pIRES2-EGFP/Ig leader-Amyloid β 1-42 (pIRES2-EGFP/IgL-Aβ) was first prepared. The APL sequence was excised by cleavage at the restriction enzyme sites (Nhe I, Xho I) added to the both ends of this sequence, while the IgL sequence was excised from the cloning vector with the same restriction enzyme pair. pIRES2-EGFP/Aβ and the IgL sequence were each purified from an agarose gel (MinElute Gel Extraction Kit) and then ligated together overnight at 16°C using a Ligation high kit (Toyobo, Tokyo, Japan), followed by transformation into TOP10 (Invitrogen, Tokyo, Japan) to select a clone carrying the desired sequence (i.e., an insert of the correct size) by restriction enzyme cleavage.

### (3) Recombination to replace EGFP sequence with M-CSF sequence

Next, the EGFP sequence was excised from pIRES2-EGFP/IgL-Aβ by cleavage at the restriction enzyme sites (BstX I, Not I), while the M-CSF sequence was excised from the cloning vector by restriction enzyme cleavage (BamH I, Not I). After pIRES2/IgL-Aβ and M-CSF were each purified from an agarose gel, the overhangs of both constructs were blunt-ended with KOD DNA polymerase (Toyobo, Tokyo, Japan). pIRES2/IgL-Aβ was treated with alkaline phosphatase (E. coli alkaline phosphatase, Toyobo, Tokyo, Japan) and then cleaned up (QIAquick PCR Purification Kit, Qiagen, Tokyo, Japan). These two constructs were ligated together using a Ligation high kit (Toyobo, Tokyo, Japan) to complete pIRES2/IgL-Aβ-moM-CSF.

The nucleotide sequence of the insert in pIRES2/IgL-Aβ-moM-CSF thus obtained is shown in SEQ ID NO: 23.

### 4. Preparation of pVAX1/IgL-Aβ1-42-huIgFc-huIL-4 and pVAX1/IgL-Aβ1-42-mIgFc-mIL-4 vaccines

### (1) Preparation of huIgFc-huIL-4 and mIgFc-mIL-4 constructs

To amplify huIgFc, pTarget/IgL-Aβ1-42-huIgFc was used as a template for PCR with primers which were designed to have appropriate restriction enzyme sites (Kpn I and Sal I) and to remove the stop codon.

To amplify mIgFc, cDNA derived from mRNA extracted from mouse spleens was used as a template for PCR with primers which were designed to have restriction enzyme sites (Kpn I and Sal I) and to remove the stop codon.

It should be noted that although the original IgFc sequence contains codons each encoding a cysteine residue (Cys), the primers were designed such that seven Cys residues were each replaced with Ser so as to avoid S-S linking.

To amplify huIL-4 and mIL-4, pTarget/IgL-Aβ1-42-huIL-4 and pTarget/IgL-Aβ1-42-mIL-4 were used as templates, respectively, for PCR with primers which were designed to remove their respective leader sequences (signal peptides) and to have a linker sequence (TCTTCTGGTGGTGGTGGT; SEQ ID NO: 24) on the 5'-side. In this case, each construct was amplified such that restriction enzyme sites (Sal I and Not I) were newly added and the stop codon found in the template was maintained.

The amplified gene fragments of huIL-4 and mIL-4 were cleaved at the previously added restriction enzyme sites. Pairs between huIgFc and huIL-4 and between mIgFc and mIL-4 were each ligated using a Ligation high kit (Toyobo, Tokyo, Japan) and cloned (pCR4-Blunt TOPO; Invitrogen, Tokyo, Japan), followed by sequencing to select a clone having the correct sequence of huIgFc-huIL-4 or mIgFc-mIL-4.

### (2) Construction of pVAX1/IgL-Aβ1-42-huIgFc-huIL-4 and pVAX1/IgL-Ap1-42-mIgFc-mIL-4

The huIgFc-huIL-4 or mIgFc-mIL-4 construct was excised by cleavage at the terminal restriction enzyme sites (Kpn I and Not I) and used for recombination with the huIL-4 moiety of pTarget/IgL-Aβ1-42-huIL-4, which had been excised by cleavage at the same sites. As a result, pTarget/IgL-Aβ1-42-huIgFc-huIL-4 and pTarget/IgL-Aβ1-42-mIgFc-mIL-4 were obtained. Further, IgL-Aβ1-42-huIgFc-huIL-4 and IgL-Aβ1-42-mIgFc-mIL-4 were each excised from the pTarget vector by cleavage at the terminal restriction enzyme sites (BamH I and Not I) and then ligated using a Ligation high kit (Toyobo, Tokyo, Japan) to a pVAX1 vector (Invitrogen, Tokyo, Japan) which had been treated with the same restriction enzyme pair, thereby completing pVAX1/IgL-Aβ1-42-huIgFc-huIL-4 and pVAX1/IgL-Aβ1-42-mIgFc-mIL-4. The nucleotide sequences of the inserts in pVAX1/IgL-Aβ1-42-huIgFc-huIL-4 and pVAX1/IgL-Aβ1-42-mIgFc-mIL-4 thus obtained are shown in SEQ ID NOs: 25 and 26, respectively.

### 5. Preparation of pVAX1/IgL-(Aβ1-42)x4-huIgFc-huIL-4 and pVAX1/IgL-(Aβ1-42)x4-moIgFc-mIL-4 vaccines

A construct carrying 4 repeats of the Aβ1-42 gene ((Aβ1-42)x4) was prepared by linking 4 units of Aβ1-42 via 3 linker sequences (GGTGGCGGTGGCTCG: SEQ ID NO: 27). First, two constructs, i.e., Aβ1-42+linker sequence+Aβ1-6 and Aβ37-42+linker sequence+Aβ1-42 were prepared by PCR amplification. Next, both constructs were mixed together and used as a template for PCR amplification with a sense primer designed to have a restriction enzyme site Xho I on the 5'-side of Aβ1-42 and an antisense primer designed to have a restriction enzyme site Kpn I on the 3'-side of Aβ1-42. The amplification products were electrophoresed on an agarose gel. Among bands in a ladder pattern, a band of about 560 bp corresponding to the molecular weight of (Aβ1-42)x4 was excised and purified.

The (Aβ1-42)x4 construct thus prepared was cloned and sequenced, and then excised by cleavage at the previously added restriction enzyme sites (Xho I and Kpn I) and used for recombination with the Aβ1-42 moiety of pTarget/IgL-Aβ1-42-huIgFc-huIL-4 or pTarget/IgL-Aβ1-42-mIgFc-mIL-4, which had been excised by cleavage at the same sites. As a result, pTarget/IgL-(Aβ1-42)x4-huIgFc-huIL-4 and pTarget/IgL-(Aβ1-42)x4-mIgFc-mIL-4 were obtained. Further, IgL-(Aβ1-42)x4-huIgFc-huIL-4 and IgL-(Aβ1-42)x4-mIgFc-mIL-4 were each excised from the pTarget vector by cleavage at the terminal restriction enzyme sites (BamH I and Not I) and then ligated using a Ligation high kit (Toyobo, Tokyo, Japan) to a pVAX1 vector (Invitrogen, Tokyo, Japan) which had been treated with the same restriction enzyme pair, thereby completing pVAX1/IgL-(Aβ1-42)x4-huIgFc-huIL-4 and pVAX1/IgL-(Ap1-42)x4-mIgFc-mIL-4. The nucleotide sequences of the inserts in pVAX1/IgL-(Aβ1-42)x4-huIgFc-huIL-4 and pVAX1/IgL-(Aβ1-42)x4-mIgFc-mIL-4 thus obtained are shown in SEQ ID NOs: 28 and 29, respectively.

### 6. Preparation of pVAX1/IgL-(Aβ1-42)x4-huIgFc and pVAX1/IgL-(Aβ1-42)x4-mIgFc vaccines

To amplify huIgFc and mIgFc each carrying a stop codon, pTarget/IgL-Aβ1-42-huIgFc-huIL-4 and pTarget/IgL-Aβ1-42-mIgFc-mIL-4 were used as templates, respectively, for PCR with primers which were designed to have appropriate restriction enzyme sites (Kpn I and Not I) and a stop codon. The amplification products were each cloned and sequenced, and then excised by cleavage at the restriction enzyme sites (Kpn I and Not I) and used for recombination with the huIgFc-huIL-4 moiety of pTarget/IgL-(Aβ1-42)x4-huIgFc-huIL-4 or with the mIgFc-mIL-4 moiety of pTarget/IgL-(Aβ1-42)x4-mIgFc-mIL-4, which had been excised by cleavage at the same sites. As a result, pTarget/IgL-(Aβ1-42)x4-huIgFc and pTarget/IgL-(Aβ1-42)x4-mIgFc were obtained. Further, IgL-(Aβ1-42)x4-huIgFc and IgL-(Aβ1-42)x4-mIgFc were each excised from the pTarget vector by cleavage at the terminal restriction enzyme sites (BamH I and Not I) and then ligated using a Ligation high kit (Toyobo, Tokyo, Japan) to a pVAX1 vector (Invitrogen, Tokyo, Japan) which had been treated with the same restriction enzyme pair, thereby completing pVAX1/IgL-(Aβ1-42)x4-huIgFc and pVAX1/IgL-(Aβ1-42)x4-mIgFc. The nucleotide sequences of the inserts in pVAXI/IgL-(Aβ1-42)x4-huIgFc and pVAX1/IgL-(Aβ1-42)x4-mIgFc thus obtained are shown in SEQ ID NOs: 30 and 31, respectively.

### [Example 2]

### Vaccination test

### 1. Vaccination test with pTarget/IgL-Aβ1-42-mIL-4 and pIRES2/IgL-Aβ1-42-moM-CSF

### (1) Materials and Methods

In the vaccination test, model mice of Alzheimer's disease were used. These model mice were obtained from the Jackson Laboratory, USA. The vaccines used were pTarget/IgL-Aβ1-42-mIL-4 (also referred to as "Aβ-IL4 vaccine"), which was prepared by integrating mouse IL-4 into the plasmid, and pIRES2/IgL-Aβ-42-moM-CSF (also referred to as "M-CSF vaccine"), which was prepared by integrating mouse M-CSF into the plasmid.

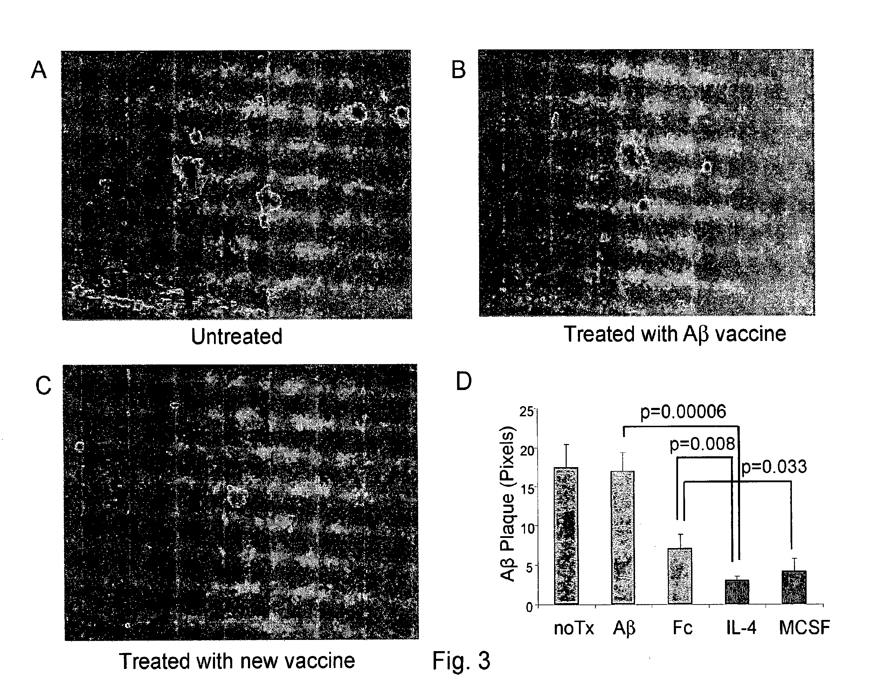

The model mice at 4 months of age were vaccinated (100 µg) once per two weeks by intramuscular injection, and the effect of eliminating deposited Aβ was observed at 10 months of age (Figure 3). First, the mice were sacrificed under deep anesthesia, and their cerebrums were excised and fixed with 4% paraformaldehyde. The fixed brains were dehydrated, embedded in paraffin, and then sliced into thin sections. The sections were deparaffinized and then immunostained with anti-Aβ antibody (Figures 3A to 3C). Ten microscopic fields of the brain cortex and hippocampus were randomly photographed from each stained section, and quantified as the total area of Aβ deposition by using NIH image software (Figure 3D).

The total amount and properties of antibody in the vaccinated animals were analyzed by ELISA and Aβ affinity assay, respectively. ELISA was performed in a standard manner, while the Aβ affinity assay was performed as follows. A test plasma was serially diluted and the resulting respective samples were contacted with cerebral sections of each model mouse to determine the presence or absence of sample binding in an immunohistological manner. The maximum dilution factor giving a positive result was defined as Aβ affinity activity of the sample.

### (2) Results

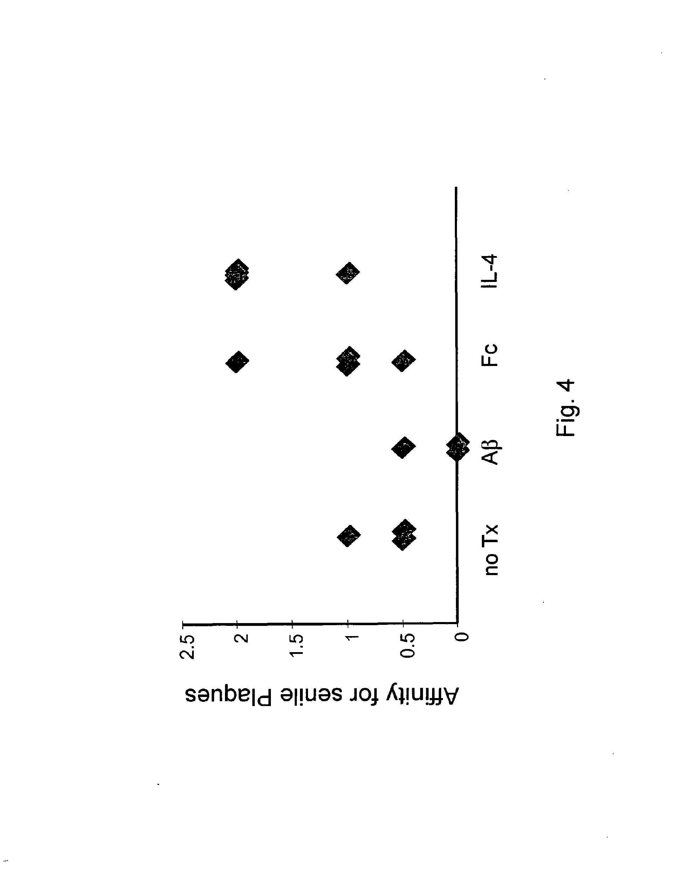

The results obtained are shown in Figures 3 and 4. In Figures 3D and 4, "noTx" represents the untreated group, "Aβ" represents the group receiving Aβ vaccination, and "Fc" represents the group receiving Aβ-Fc vaccination. Likewise, "IL-4" represents the group receiving Aβ-IL4 vaccination, and "MCSF" represents the group receiving M-CSF vaccination.

In Figure 3, when compared to the untreated group (Figure 3A), the group receiving existing vaccination (Aβ vaccine, Figure 3B) shows an Aβ-eliminating effect, which is not so remarkable. In contrast, the reference vaccine (Aβ-IL4 vaccine) was clearly found to have a stronger Aβ-eliminating effect (Figure 3C). These results were quantified and analyzed, indicating that the reference Aβ-IL4 vaccine had a significantly strong Aβ-eliminating effect, when compared to the existing vaccines (Aβ vaccine, Aβ-Fc vaccine (IgL-Aβ-Fc)) (Figure 3D). Moreover, the reference M-CSF vaccine was also found to have a remarkable Aβ-eliminating effect (Figure 3D).

Figure 4 indicated that the mice receiving reference Aβ-IL4 vaccination showed increased levels of antibody with high affinity for Aβ deposition (senile plaques), although there was no significant difference over the control group in their anti-Aβ-IgG levels (Figure 4). The nature of this induced antibody would support the high Aβ-eliminating effect produced by the reference Aβ-IL4 vaccine.

### 2. Vaccination test with pVAX1/IgL-(Aβ1-42)x4-huIgFc-huIL-4 (YM3711) and others (1) Materials and Methods

pVAX1/IgL-Aβ1-42-huIgFc-huIL-4 (Figure 5), pVAX1/IgL-Aβ1-42-mIgFc-mIL-4 (Figure 6), pVAX1/IgL-(Aβ1-42)x4-huIgFc-huIL-4 (Figure 7), pVAX1/IgL-(Aβ1-42)x4-mIgFc-mIL-4 (Figure 8), pVAX1/IgL-(Aβ1-42)x4-huIgFc (Figure 9) and pVAX1/IgL-(Aβ1-42)x4-mIgFc (Figure 10) were newly constructed and used in the vaccination test.

In the vaccination test, model mice of Alzheimer's disease were used. These model mice were obtained from the Jackson Laboratory, USA.

The vaccines used for mice were pVAX1/IgL-Aβ1-42-mIgFc-mIL-4, pVAX1/IgL-(Aβ1-42)x4-mIgFc-mIL-4 and pVAX1/IgL-(Aβ1-42)x4-mIgFc. The vaccines used for rabbits were pVAX1/IgL-Aβ1-42-huIgFc-huIL-4, pVAX1/IgL-(Aβ1-42)x4-huIgFc-huIL-4 and pVAX1/IgL-(Aβ1-42)x4-mIgFc.

First, the above vaccines were each introduced into cultured cells. The cells were incubated for 3 days and then analyzed by sandwich ELISA to determine the amounts of Aβ present within the cells and in the culture supernatant. The cultured cells used were HEK392 cells.

Next, the model mice at 10 months of age were vaccinated (100 µg) once a week by intramuscular injection, and blood was sampled over time from their orbita. Plasma was separated and analyzed by ELISA to determine the titer of anti-Aβ antibody. In the same manner, rabbits were also vaccinated and measured for antibody titer in their plasma.

The model mice were sacrificed at 12 months of age to study the effect of vaccination on elimination of Aβ deposited in the brain. First, cervical dislocation was performed under deep anesthesia, and cerebrums were excised and fixed with 4% paraformaldehyde. The fixed brains were dehydrated, embedded in paraffin, and then sliced into thin sections. The sections were deparaffinized and then immunostained with anti-Aβ antibody. Ten microscopic fields of the brain cortex were randomly photographed from each stained section, and quantified as the total area of Aβ deposition by using NIH image software.

### (2) Results

The representative results are shown in Figures 11 to 13.

In Figure 11, "IgL-Aβ-IL4" represents pVAX1/IgL-Aβ1-42-mIL-4, and "IgL-Aβ-Fc-IL4" represents pVAX1/IgL-Aβ1-42-mIgFc-mIL-4. Likewise, "cell pellet" represents the cell pellet fraction, and "sup." represents the culture supernatant fraction.

Upon introduction into cultured cells, the vaccine carrying immunoglobulin Fc sequence ("IgL-Aβ-Fc-IL4") showed a several-fold or more increase in Aβ production within the cultured cells ("cell pellet"), when compared to the Fc sequence-free vaccine ("IgL-Aβ-IL4"). Moreover, the Fc sequence-free vaccine caused little Aβ secretion into the culture supernatant, whereas the Fc sequence-carrying vaccine was found to cause remarkable secretion ("sup.").

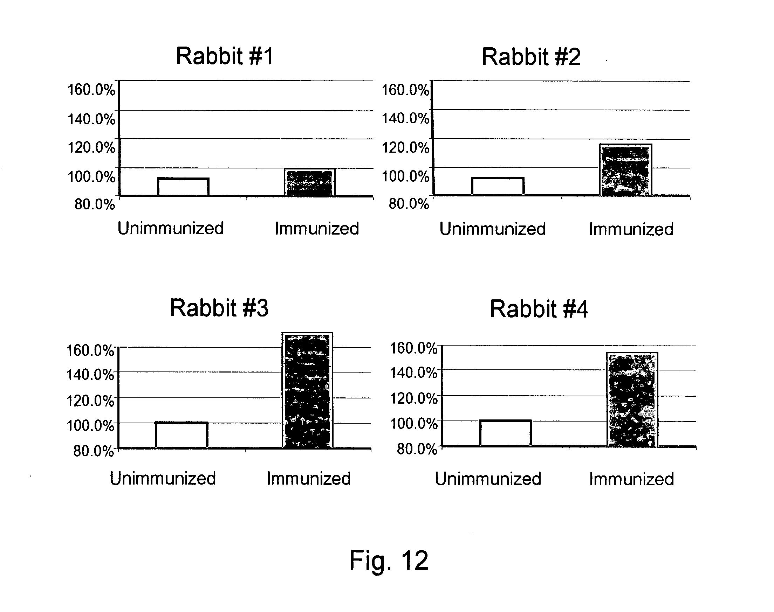

When rabbits were administered with pVAX1/IgL-Aβ1-42-huIgFc-huIL-4 (hereinafter also referred to as "Aβ(1-42)x1 vaccine") and pVAX1/IgL-(Aβ1-42)x4-huIgFc-huIL-4 (hereinafter also referred to as "YM3711"), not only the Aβ(1-42)x1 vaccine carrying no repeat of Aβ1-42, but also YM3711 carrying repeats of Aβ1-42 caused significant induction of anti-Aβ antibody (Figure 12). Moreover, the rabbits administered with YM3711 carrying repeats of Aβ1-42 ("Rabbits #3 and #4" in Figure 12) showed higher antibody titers than the rabbits administered with the Aβ(1-42)x1 vaccine carrying no repeat of Aβ1-42 ("Rabbits #1 and #2" in Figure 12) (Figure 12). This result indicates that the vector of the present invention is useful as an inducer of anti-Aβ antibody.

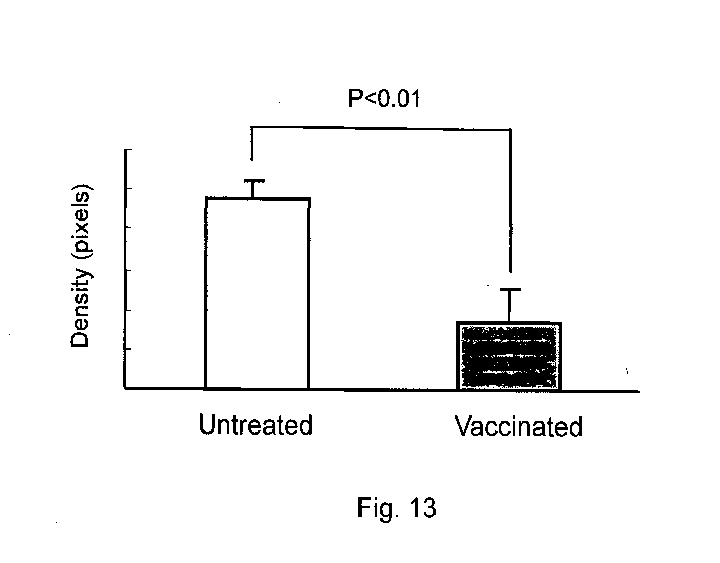

In the quantification of brain Aβ deposition, the mice administered with pVAX1/IgL-(Aβ1-42)x4-mIgFc-mIL-4 clearly showed a remarkable Aβ-eliminating effect, in comparison with the untreated group (Figure 13).

Until now, some attempts have been made to treat Alzheimer model mice with a vaccine prepared by introducing a gene for Aβ1-42, IL-4 or tissue plasminogen activator into a plasmid vector. However, such a DNA vaccine alone was not sufficient to induce a decrease in brain Aβ levels. This would be because the structure of these vaccines has a defect in the mechanism for elevating anti-Aβ antibody levels. As shown above, the vaccines prepared by the inventors of the present invention were found to be effective by themselves and to produce a higher Aβ-eliminating effect than other existing vaccines. In particular, the human and mouse types of pVAX1/IgL-(Aβ1-42)x4-IgFc-IL-4 vaccines were shown to have an extremely high Aβ-eliminating effect.

### 3. Study on pVAX1/IgL-(Aβ1-42)x4-huIgFc-huIL-4 (YM3711) for its ability to induce various antibodies

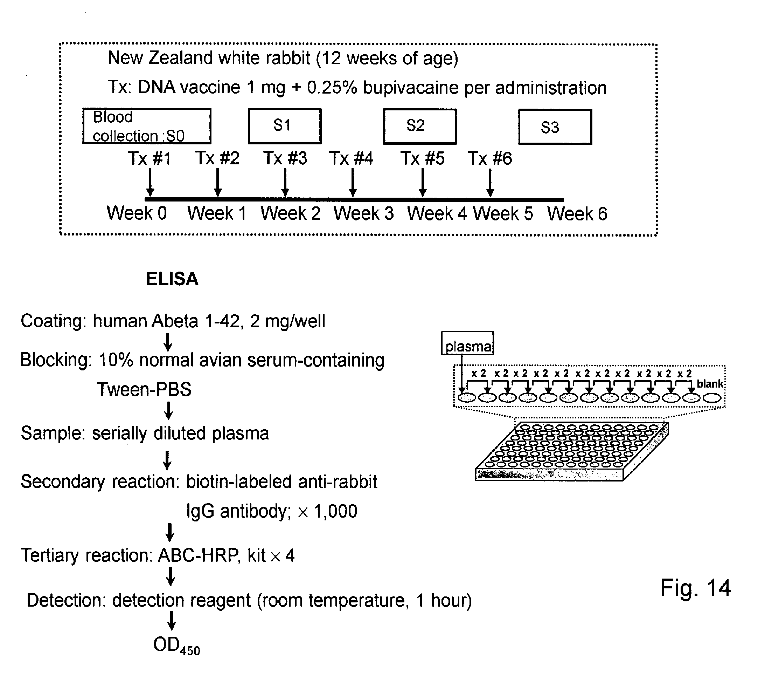

The inventors of the present invention studied pVAX1/IgL-(Aβ1-42)x4-huIgFc-huIL-4 (YM3711) for its ability to induce various antibodies in the same manner as used in the vaccination test in 2 above. More specifically, rabbits were injected six times (once a week for 6 weeks) with 1 mg of YM3711 by the intramuscular route, and blood was sampled over time. Plasma was separated and analyzed by ELISA to determine the titer of anti-Aβ antibody in the plasma, which was then compared with the unimmunized plasma. The same test was also repeated using an existing vaccine (pVAX1/IgL-Aβ1-42-huIgFc), and the degree of antibody titer in this case was compared with that of YM3711. Figure 14 shows the schedule of DNA vaccination and procedures for antibody titer assay in rabbits.

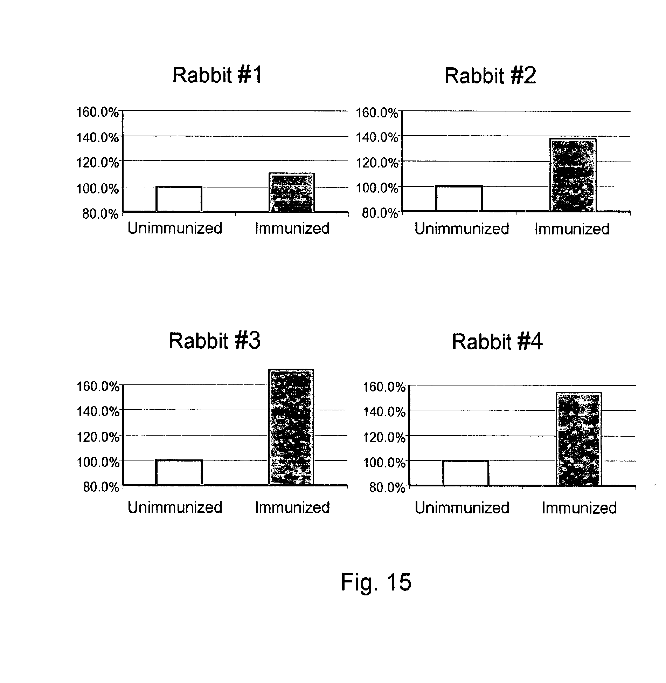

As a result, the rabbits administered with YM3711 ("Rabbits #3 and #4" in Figure 15) were found to show a higher increase in the antibody titer of anti-Aβ1-42 antibody than the rabbits administered with the existing vaccine ("Rabbits #1 and #2" in Figure 15) (Figure 15). Table 1 below shows the % increase in antibody titer after immunization with YM3711.
[Table 1]

**Table 1 Increase (%) in antibody titer after immunization**

| Anti-Aβ1-42 antibody | | (%) |
|---|---|---|
| | Rabbit #1 | Rabbit #2 |
| Existing vaccine | 12 | 39 |

| | Rabbit #3 | Rabbit #4 |
|---|---|---|
| YM3711 | 68 | 58 |

As shown in Table 1, the rabbits administered with the existing vaccine ("Rabbits #1 and #2" in Table 1) showed an increase of 12% to 39% when compared to before immunization. In contrast, the rabbits administered with YM3711 ("Rabbits #3 and #4" in Table 1) showed an increase as high as 58% to 68%. This indicates that YM3711 causes remarkable induction of anti-Aβ1-42 antibody in rabbits, in comparison with the existing vaccine.

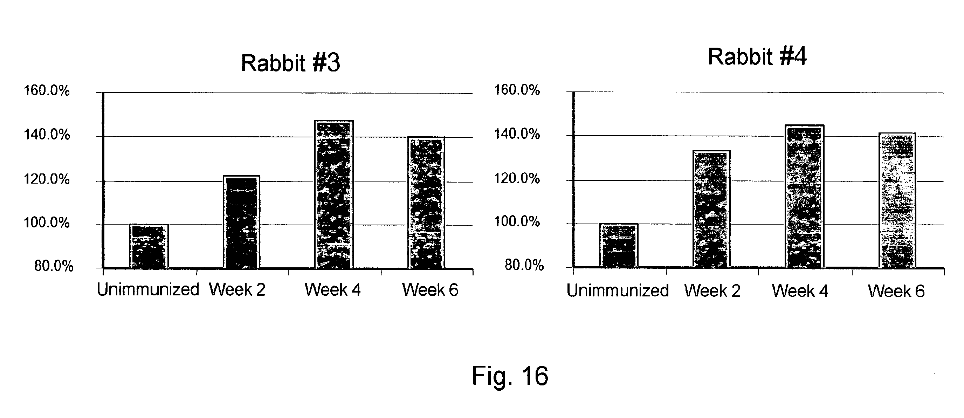

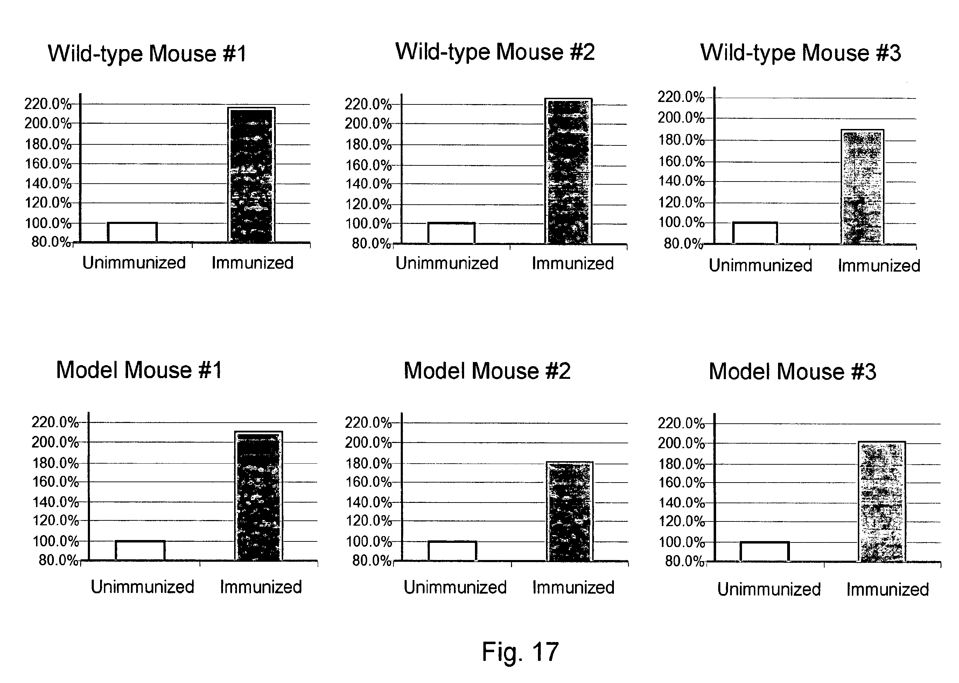

The antibody titer of anti-Aβ1-42 antibody started to increase at 2 weeks after immunization and was then maintained at a high level (Figure 16). Moreover, not only the rabbits, but also wild-type or model mice showed an increase in antibody titer upon DNA vaccination (Figure 17). Table 2 below shows the % increase in antibody titer after wild-type and model mice were each immunized with YM3711.
[Table 2]

**Table 2 Anti-Aβ antibody-inducing effect of YM3711 in mice**

| | | | (%) |
|---|---|---|---|
| | #1 | #2 | #3 |
| Wild-type mouse | 119 | 124 | 91 |
| Model mouse | 111 | 81 | 100 |

As shown in Table 2, YM3711 was found to cause an increase of 91 % to 124% in the wild-type mice and 81% to 111% in the model mice. This indicates that YM3711 also causes remarkable induction of anti-Aβ1-42 antibody in both wild-type and model mice.

The inventors of the present invention further conducted the same test in monkeys, which are most important in drug development. More specifically, male cynomolgus monkeys at 3.5 to 4.5 years of age (3.0 to 4.0 kg) were injected with 0.5 mg/kg of YM3711 once a week by the intramuscular route, and blood was collected before immunization and after 2 and 4 weeks. Plasma fractions of these blood samples were used in ELISA using the Aβ1-42 peptide as an antigen to determine their anti-Aβ antibody titer. In this test, the antibody titer before immunization was set to 100%, and % elevation (% increase) of antibody titer was calculated for each animal.

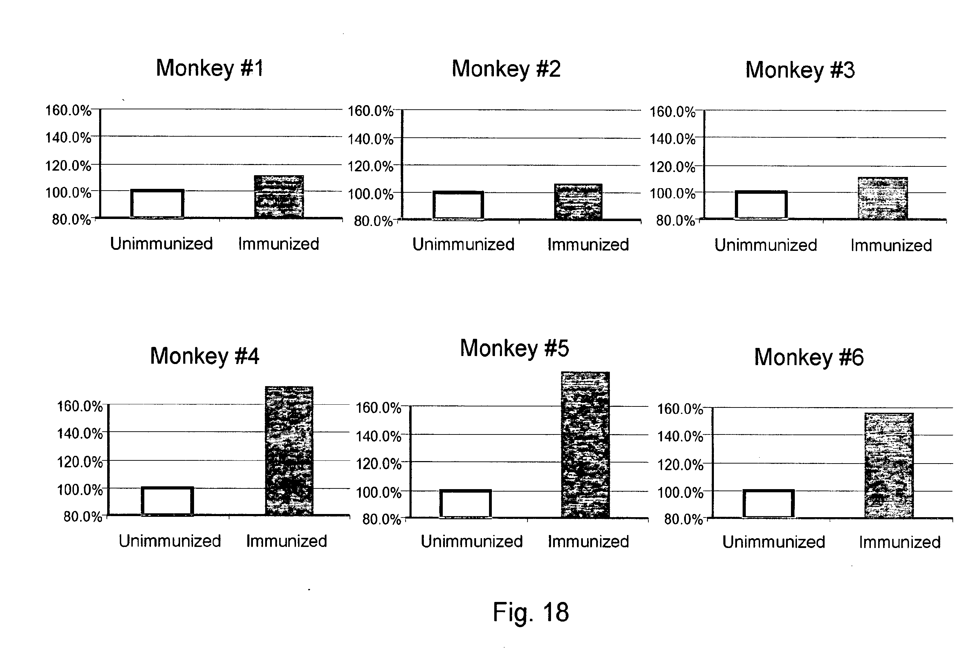

As a result, the monkeys administered with YM3711 ("Monkeys #4 to #6" in Figure 18) were found to show a remarkably higher increase in the antibody titer of anti-Aβ1-42 antibody than the monkeys administered with the existing vaccine (pVAX1/IgL-Aβ1-42-huIgFc) ("Monkeys #1 to #3" in Figure 18) (Figure 18). Table 3 below shows the % increase in antibody titer after immunization with YM3711.
[Table 3]

**Table 3 Increase (%) in antibody titer after immunization**

| Anti-Aβ1-42 antibody | | | (%) |
|---|---|---|---|
| | Monkey #1 | Monkey #2 | Monkey #3 |
| Existing vaccine | 12 | 8 | 12 |

| | Monkey #4 | Monkey #5 | Monkey #6 |
|---|---|---|---|
| YM3711 | 75 | 81 | 58 |

As shown in Table 3, the monkeys administered with the existing vaccine ("Monkeys #1 to #3" in Table 3) showed an increase of 8% to 12% when compared to before immunization. In contrast, the monkeys administered with YM3711 ("Monkeys #4 to #6" in Table 3) showed an increase as high as 58% to 81%. This indicates that YM3711 causes remarkable induction of anti-Aβ1-42 antibody in monkeys, in comparison with the existing vaccine.

Moreover, in comparison with the test in rabbits (Figure 15 and Table 1), the monkeys showed a remarkable difference between the increase in antibody titer after immunization with the existing vaccine and the increase in antibody titer after immunization with YM3711. This indicates that YM3711 is a vaccine or an inducer of anti-Aβ1-42 antibody, which is more compatible than the existing vaccine with animals closer to humans.

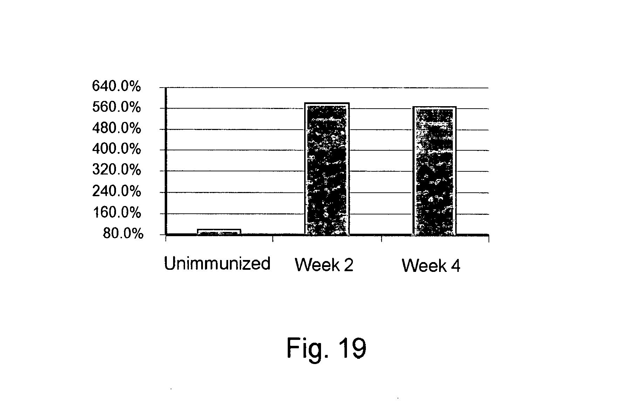

In the monkeys, the antibody titer of anti-Aβ1-42 antibody started to increase at 2 weeks after immunization and was then maintained at a high level, which indicated a sufficient increase in anti-Aβ antibody levels until 4 weeks after immunization (Figure 19).

Moreover, to study the ability of YM3711 to induce various antibodies, the same procedure as used above in antibody titer assay for anti-Aβ1-42 antibody was repeated to determine the antibody titers of other antibodies, i.e., antibody against pEAβ3-42 having strong neurotoxicity, as well as antibodies against ABri and ADan having high amyloid aggregation propensity although their amino acid sequences are completely different from that of Aβ. Antibody titer assay was performed with rabbits and monkeys.

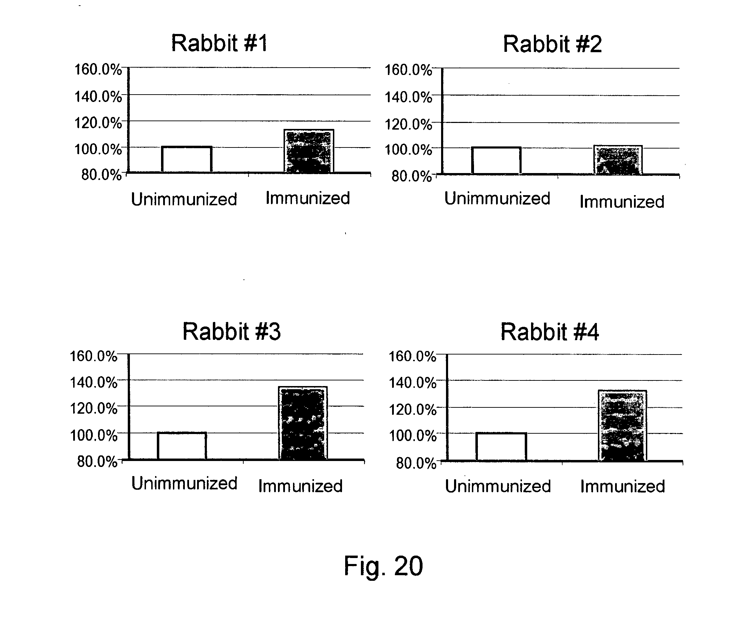

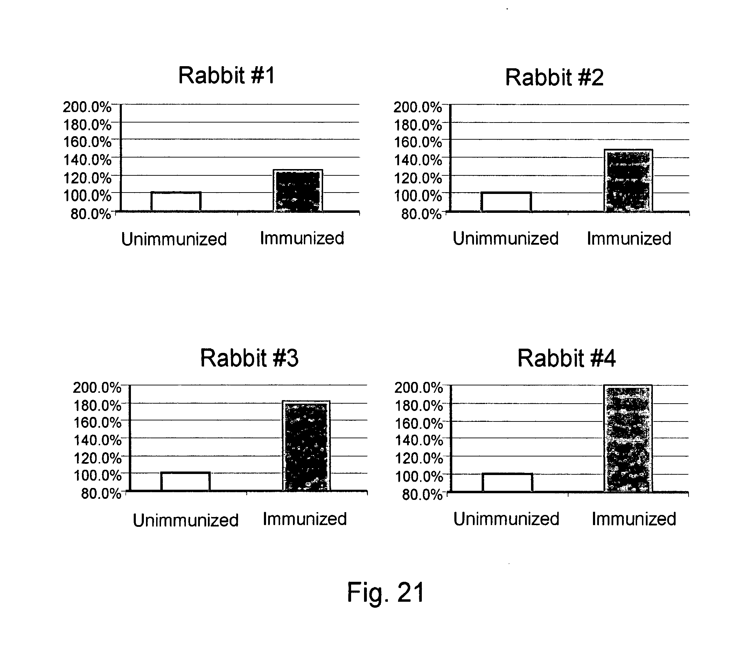

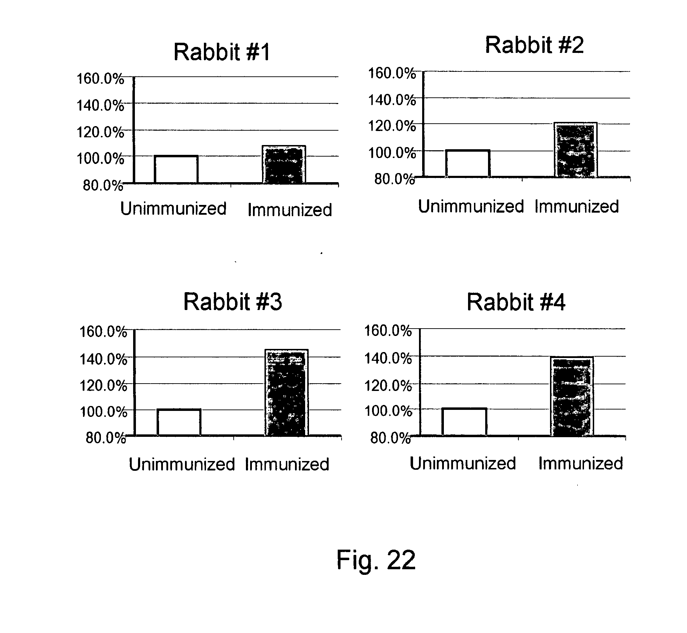

The results obtained with rabbits are shown in Figures 20 to 22. Figure 20 shows the anti-pEAβ3-42 antibody-inducing effect produced by YM3711, Figure 21 shows the anti-ABri antibody-inducing effect produced by YM3711, and Figure 22 shows the anti-ADan antibody-inducing effect produced by YM3711. In Figures 20 to 22, "Rabbits #1 and #2" show the results in rabbits administered with the existing vaccine (pVAX1/IgL-Aβ1-42-huIgFc), while "Rabbits #3 and #4" show the results in rabbits administered with YM3711.

These results indicated that YM3711 induced anti-pEAβ3-42 antibody, anti-ABri antibody and anti-ADan antibody in the rabbits administered therewith. Table 4 below shows the % increase in antibody titer after immunization with YM3711.
[Table 4]

**Table 4 Increase (%) in antibody titer after immunization**

| Anti-pEAβ3-42 antibody | | (%) |
|---|---|---|
| | Rabbit #1 | Rabbit #2 |
| Existing vaccine | 18 | 1 |

| | Rabbit #3 | Rabbit #4 |
|---|---|---|
| YM3711 | 38 | 37 |
| | | |

| Anti-ABri antibody | | (%) |
|---|---|---|
| | Rabbit #1 | Rabbit #2 |
| Existing vaccine | 26 | 50 |

| | Rabbit #3 | Rabbit #4 |
|---|---|---|
| YM3711 | 81 | 100 |
| | | |

| Anti-ADan antibody | | (%) |
|---|---|---|
| | Rabbit #1 | Rabbit #2 |
| Existing vaccine | 8 | 20 |

| | Rabbit #3 | Rabbit #4 |
|---|---|---|
| YM3711 | 43 | 39 |

As shown in Table 4, the rabbits administered with the existing vaccine ("Rabbits #1 and #2" in Table 4) showed an increase of 1% to 18% for anti-pEAβ3-42 antibody, 26% to 50% for anti-ABri antibody, and 8% to 20% for anti-ADan antibody, when compared to before immunization. In contrast, the rabbits administered with YM3711 ("Rabbits #3 and #4" in Table 4) showed an increase of 37% to 38% for anti-pEAβ3-42 antibody, 81% to 100% for anti-ABri antibody, and 39% to 43% for anti-ADan antibody. This indicates that YM3711 causes remarkable induction of anti-pEAβ3-42 antibody, anti-ABri antibody and anti-ADan antibody in rabbits, in comparison with the existing vaccine.

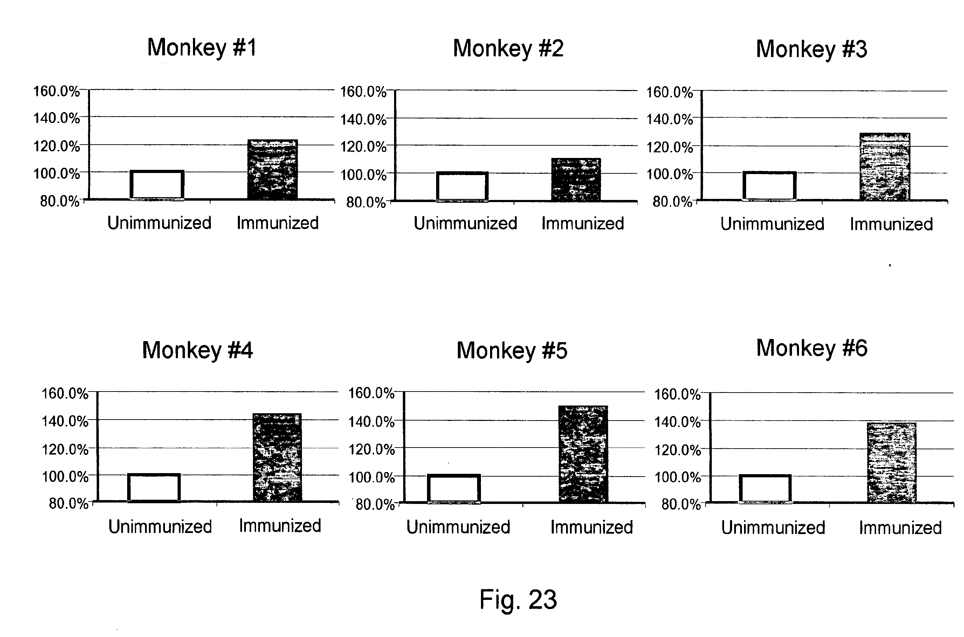

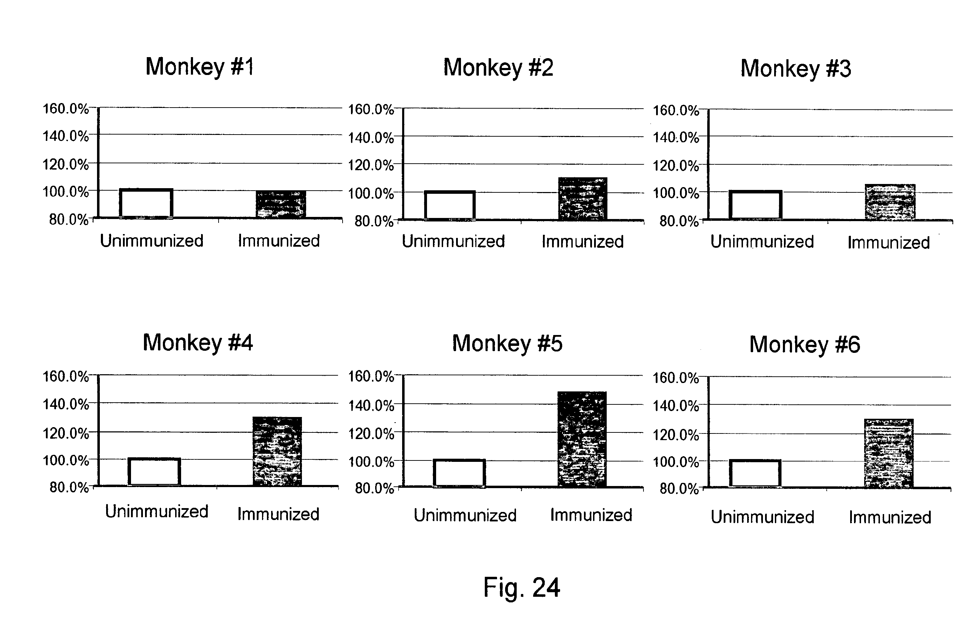

The results obtained with monkeys are shown in Figures 23 and 24. Figure 23 shows the anti-pEAβ3-42 antibody-inducing effect produced by YM3711, and Figure 24 shows the anti-ADan antibody-inducing effect produced by YM3711. In Figures 23 and 24, "Monkeys #1 to #3" show the results in monkeys administered with the existing vaccine (pVAX1/IgL-Aβ1-42-huIgFc), while "Monkeys #4 to #6" show the results in monkeys administered with YM3711.

These results indicated that YM3711 induced anti-pEAβ3-42 antibody and anti-ADan antibody in the monkeys administered therewith. Table 5 below shows the % increase in antibody titer after immunization with YM3711.
[Table 5]

**Table 5 Increase (%) in antibody titer after immunization**

| Anti-pEAβ3-42 antibody | | | (%) |
|---|---|---|---|
| | Monkey #1 | Monkey #2 | Monkey #3 |
| Existing vaccine | 24 | 10 | 30 |

| | Monkey #4 | Monkey #5 | Monkey #6 |
|---|---|---|---|
| YM3711 | 43 | 50 | 39 |
| | | | |

| Anti-ADan antibody | | | (%) |
|---|---|---|---|
| | Monkey #1 | Monkey #2 | Monkey #3 |
| Existing vaccine | 0 | 9 | 4 |

| | Monkey #4 | Monkey #5 | Monkey #6 |
|---|---|---|---|
| YM3711 | 31 | 49 | 30 |

As shown in Table 5, the monkeys administered with the existing vaccine ("Monkeys #1 to #3" in Table 5) showed an increase of 10% to 30% for anti-pEAβ3-42 antibody and 0% to 9% for anti-ADan antibody, when compared to before immunization. In contrast, the monkeys administered with YM3711 ("Monkeys #4 to #6" in Table 5) showed an increase of 39% to 50% for anti-pEAβ3-42 antibody and 30% to 49% for anti-ADan antibody. This indicates that YM3711 causes remarkable induction of anti-pEAβ3-42 antibody and anti-ADan antibody in monkeys, in comparison with the existing vaccine.

Further studies were conducted to determine whether anti-IgFc antibody and anti-IL-4 antibody were induced upon YM3711 immunization, there was no induction of these antibodies. The reason is not clear why antibody against repeats of Aβ is induced, whereas no antibody is induced against the IgFc or IL-4 moiety. However, human IgFc and IL-4 molecules are similar to those of monkeys and hence are recognized as self-components by the monkey's body, so that antibodies against these molecules would be difficult to induce. In contrast, Aβ molecules, which are of self origin, abnormally aggregate and hence the body will recognize the aggregate as a foreign substance to cause antibody production.

This finding is important for clinical use of the vaccine in avoiding unwanted immune responses and in enhancing vaccine safety.

In view of the foregoing, the vaccines of the present invention were found not only to induce a wide range of antibodies against various neurotoxic molecules, including predominantly Aβ, in Alzheimer's disease, but also to eliminate Aβ deposition. No DNA vaccine showing such a mechanism of action has been reported in the past.

### INDUSTRIAL APPLICABILITY

The present invention provides DNA vaccines for prevention or treatment of Alzheimer's disease. The vaccines of the present invention remarkably suppress Aβ accumulation and hence are useful as pharmaceutical compositions for treatment or prevention of Alzheimer's disease.

### Sequence Listing Free Text

SEQ ID NO: 1: synthetic DNA
SEQ ID NO: 2: synthetic peptide
SEQ ID NO: 3: synthetic DNA
SEQ ID NO: 4: synthetic peptide
SEQ ID NO: 5: synthetic DNA
SEQ ID NO: 6: synthetic peptide
SEQ ID NO: 7: synthetic DNA
SEQ ID NO: 8: synthetic peptide
SEQ ID NO: 9: synthetic DNA
SEQ ID NO: 10: synthetic peptide
SEQ ID NO: 11: synthetic DNA
SEQ ID NO: 12: synthetic peptide
SEQ ID NO: 13: synthetic DNA
SEQ ID NO: 14: synthetic peptide
SEQ ID NO: 15: synthetic DNA
SEQ ID NO: 16: synthetic peptide
SEQ ID NO: 21: synthetic DNA
SEQ ID NO: 22: synthetic DNA
SEQ ID NO: 23: synthetic DNA
SEQ ID NO: 24: synthetic DNA
SEQ ID NO: 25: synthetic DNA
SEQ ID NO: 26: synthetic DNA
SEQ ID NO: 27: synthetic DNA
SEQ ID NO: 28: synthetic DNA
SEQ ID NO: 29: synthetic DNA
SEQ ID NO: 30: synthetic DNA
SEQ ID NO: 31: synthetic DNA

## Claims

1. A recombinant vector, which comprises repeats of DNA encoding Aβ1-42, a DNA encoding an immunoglobulin Fc sequence and a DNA encoding interleukin-4.

2. The recombinant vector of claim 1, which comprises 2 to 4 repeats of DNA encoding Aβ1-42.

3. The recombinant vector of claim 2, wherein the immunoglobulin Fc sequence is human and the DNA encoding interleukin-4 is human.

4. A DNA vaccine which comprises the recombinant vector according to one of claims 1 to 3.

5. The DNA vaccine of claim 4, for use in the prevention or treatment of Alzheimer's disease.

6. An inducer of anti-Aβ antibody, which comprises the recombinant vector according to claim 1.

## Patentansprüche

1. Ein rekombinanter Vektor, der Repeats von Aβ1-42 kodierender DNS, eine Sequenz von Immunoglobulin Fc kodierende DNS und Interleukin-4 kodierende DNS umfasst.

2. Der rekombinante Vektor von Anspruch 1, der 2 bis 4 Repeats von Aβ1-42 kodierender DNS umfasst.

3. Der rekombinante Vektor von Anspruch 2, in dem die Sequenz von Immunoglobulin Fc und die Interleukin-4 kodierende DNS menschlich sind.

4. Ein DNS-Impfstoff, der den rekombinanten Vektor gemäss einem der Ansprüche 1 bis 3 umfasst.

5. Der DNS-Impfstoff von Anspruch 4, zur Verwendung in der Prävention oder Behandlung der Alzheimer-Krankheit.

6. Ein Induzierer der Anti-Aβ-Antikörper, der den rekombinanten Vektor gemäss Anspruch 1 umfasst.

## Revendications

1. Vecteur recombiné qui comprend des répétitions d'ADN codant pour Aβ1-42, un ADN codant pour une séquence d'immunoglobuline Fc et un ADN codant pour l'interleukine 4.

2. Vecteur recombiné conforme à la revendication 1, comprenant 2 à 4 répétitions d'ADN codant pour Aβ1-42.

3. Vecteur recombiné conforme à la revendication 2, dans lequel la séquence d'immunoglobuline Fc est une séquence d'immunoglobuline Fc humaine et l'ADN codant pour l'interleukine 4 est un ADN humain.

4. Vaccin à ADN qui comprend le vecteur recombiné conforme à l'une des revendications 1 à 3.

5. Vaccin à ADN conforme à la revendication 4, destiné à être utilisé pour la prévention ou le traitement de la maladie d'Alzheimer.

6. Inducteur d'anticorps anti-Aβ renfermant le vecteur recombiné conforme à la revendication 1.


### SEQUENCE LISTING

<110> Tokyo Metropolitan Organization for Medical Research
<120> DNA vaccine against Alzheimer disease
<130> PCT10-0010
<150> JP 2009-075832
   <151> 2009-03-26
<160> 33
<170> PatentIn version 3.4
<210> 1
   <211> 129
   <212> DNA
   <213> Artificial
<220>
   <223> synthetic DNA
<220>
   <221> CDS
   <222> (1)..(129)
<400> 1
   gat gca gaa ttc cga cat gac tca gga tat gaa gtt cat cat caa aaa         48
   Asp Ala Glu Phe Arg His Asp Ser Gly Tyr Glu Val His His Gln Lys
   1               5                   10                  15
   ttg gtg ttc ttt gca gaa gat gtg ggt tca aac aaa ggt gca atc att         96
   Leu Val Phe Phe Ala Glu Asp Val Gly Ser Asn Lys Gly Ala Ile Ile
               20                  25                  30
   gga ctc atg gtg ggc ggt gtt gtc ata gcg aca                            129
   Gly Leu Met Val Gly Gly Val Val Ile Ala Thr
           35                  40
<210> 2
   <211> 43
   <212> PRT
   <213> Artificial
<220>
   <223> Synthetic Construct
<400> 2
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40
<210> 3
   <211> 60
   <212> DNA
   <213> Artificial
<220>
   <223> synthetic DNA
<220>
   <221> CDS
   <222> (1)..(60)
<400> 3
   gat gca gaa ttc cga cat gac tca gga tat gaa gtt cat cat caa aaa         48
   Asp Ala Glu Phe Arg His Asp Ser Gly Tyr Glu Val His His Gln Lys
   1               5                   10                  15
   ttg gtg ttc ttt                                                         60
   Leu Val Phe Phe
               20
<210> 4
   <211> 20
   <212> PRT
   <213> Artificial
<220>
   <223> Synthetic Construct
<400> 4
   Asp Ala Glu Phe Arg His Asp Ser Gly Tyr Glu Val His His Gln Lys
   1               5                   10                  15
   Leu Val Phe Phe
               20
<210> 5
   <211> 120
   <212> DNA
   <213> Artificial
<220>
   <223> synthetic DNA
<220>
   <221> CDS
   <222> (1)..(120)
<400> 5
   gat gca gaa ttc cga cat gac tca gga tat gaa gtt cat cat caa aaa       48
   Asp Ala Glu Phe Arg His Asp Ser Gly Tyr Glu Val His His Gln Lys
   1               5                   10                  15
   ttg gtg ttc ttt gca gaa gat gtg ggt tca aac aaa ggt gca atc att       96
   Leu Val Phe Phe Ala Glu Asp Val Gly Ser Asn Lys Gly Ala Ile Ile
               20                  25                  30
   gga ctc atg gtg ggc ggt gtt gtc                                       120
   Gly Leu Met Val Gly Gly Val Val
           35                  40
<210> 6
   <211> 40
   <212> PRT
   <213> Artificial
<220>
   <223> Synthetic Construct
<400> 6
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 7
   <211> 126
   <212> DNA
   <213> Artificial
<220>
   <223> synthetic DNA
<220>
   <221> CDS
   <222> (1)..(126)
<400> 7
   gat gca gaa ttc cga cat gac tca gga tat gaa gtt cat cat caa aaa      48
   ttg gtg ttc ttt gca gaa gat gtg ggt tca aac aaa ggt gca atc att      96
   gga ctc atg gtg ggc ggt gtt gtc ata gcg                              126
<210> 8
   <211> 42
   <212> PRT
   <213> Artificial
<220>
   <223> Synthetic Construct
<400> 8
<210> 9
   <211> 129
   <212> DNA
   <213> Artificial
<220>
   <223> synthetic DNA
<220>
   <221> CDS
   <222> (1)..(129)
<400> 9
   gat gca gaa ttc gga cat gat tca gga ttt gaa gtc cgc cat caa aaa      48
   Asp Ala Glu Phe Gly His Asp Ser Gly Phe Glu Val Arg His Gln Lys
   1               5                   10                  15
   ctg gtg ttc ttt gct gaa gat gtg ggt tcg aac aaa ggc gcc atc atc      96
   Leu Val Phe Phe Ala Glu Asp Val Gly Ser Asn Lys Gly Ala Ile Ile
               20                  25                  30
   gga ctc atg gtg ggc ggc gtt gtc ata gca acc                          129
   Gly Leu Met Val Gly Gly Val Val Ile Ala Thr
           35                  40
<210> 10
   <211> 43
   <212> PRT
   <213> Artificial
<220>
   <223> Synthetic Construct
<400> 10
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40
<210> 11
   <211> 60
   <212> DNA
   <213> Artificial
<220>
   <223> synthetic DNA
<220>
   <221> CDS
   <222> (1)..(60)
<400> 11
   gat gca gaa ttc gga cat gat tca gga ttt gaa gtc cgc cat caa aaa      48
   Asp Ala Glu Phe Gly His Asp Ser Gly Phe Glu Val Arg His Gln Lys
   1               5                   10                  15
   ctg gtg ttc ttt                                                       60
   Leu Val Phe Phe
               20
<210> 12
   <211> 20
   <212> PRT
   <213> Artificial
<220>
   <223> Synthetic Construct
<400> 12
   Asp Ala Glu Phe Gly His Asp Ser Gly Phe Glu Val Arg His Gln Lys
   1               5                   10                  15
   Leu Val Phe Phe
               20
<210> 13
   <211> 120
   <212> DNA
   <213> Artificial
<220>
   <223> synthetic DNA
<220>
   <221> CDS
   <222> (1)..(120)
<400> 13
   gat gca gaa ttc gga cat gat tca gga ttt gaa gtc cgc cat caa aaa      48
   Asp Ala Glu Phe Gly His Asp Ser Gly Phe Glu Val Arg His Gln Lys
   1               5                   10                  15
   ctg gtg ttc ttt gct gaa gat gtg ggt tcg aac aaa ggc gcc atc atc      96
   Leu Val Phe Phe Ala Glu Asp Val Gly Ser Asn Lys Gly Ala Ile Ile
               20                  25                  30
   gga ctc atg gtg ggc ggc gtt gtc                                      120
   Gly Leu Met Val Gly Gly Val Val
           35                  40
<210> 14
   <211> 40
   <212> PRT
   <213> Artificial
<220>
   <223> Synthetic Construct
<400> 14
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 15
   <211> 126
   <212> DNA
   <213> Artificial
<220>
   <223> synthetic DNA
<220>
   <221> CDS
   <222> (1)..(126)
<400> 15
   gat gca gaa ttc gga cat gat tca gga ttt gaa gtc cgc cat caa aaa        48
   Asp Ala Glu Phe Gly His Asp Ser Gly Phe Glu Val Arg His Gln Lys
   1               5                   10                  15
   ctg gtg ttc ttt gct gaa gat gtg ggt tcg aac aaa ggc gcc atc atc        96
   Leu Val Phe Phe Ala Glu Asp Val Gly Ser Asn Lys Gly Ala Ile Ile
               20                  25                  30
   gga ctc atg gtg ggc ggc gtt gtc ata gca                               126
   Gly Leu Met Val Gly Gly Val Val Ile Ala
           35                  40
<210> 16
   <211> 42
   <212> PRT
   <213> Artificial
<220>
   <223> Synthetic Construct
<400> 16
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala
           35                  40
<210> 17
   <211> 462
   <212> DNA
   <213> Homo sapiens
<400> 17
   atgggtctca cctcccaact gcttccccct ctgttcttcc tgctagcatg tgccggcaac      60
   tttgtccacg gacacaagtg cgatatcacc ttacaggaga tcatcaaaac tttgaacagc     120
   ctcacagagc agaagactct gtgcaccgag ttgaccgtaa cagacatctt tgctgcctcc     180
   aagaacacaa ctgagaagga aaccttctgc agggctgcga ctgtgctccg gcagttctac     240
   agccaccatg agaaggacac tcgctgcctg ggtgcgactg cacagcagtt ccacaggcac     300
   aagcagctga tccgattcct gaaacggctc gacaggaacc tctggggcct ggcgggcttg     360
   aattcctgtc ctgtgaagga agccaaccag agtacgttgg aaaacttctt ggaaaggcta     420
   aagacgatca tgagagagaa atattcaaag tgttcgagct ga                        462
<210> 18
   <211> 423
   <212> DNA
   <213> Mus musculus
<400> 18
   atgggtctca acccccagct agttgtcatc ctgctcttct ttctcgaatg taccaggagc      60
   catatccacg gatgcgacaa aaatcacttg agagagatca tcggcatttt gaacgaggtc     120
   acaggagaag ggacgccatg cacggagatg gatgtgccaa acgtcctcac agcaacgaag     180
   aacaccacag agagtgagct cgtctgtagg gcttccaagg tgcttcgcat attttattta     240
   aaacatggga aaactccatg cttgaagaag aactctagtg ttctcatgga gctgcagaga     300
   ctctttcggg cttttcgatg cctggattca tcgataagct gcaccatgaa tgagtccaag     360
   tccacatcac tgaaagactt cctggaaagc ctaaagagca tcatgcaaat ggattactcg     420
   tag                                                                   423
<210> 19
   <211> 1665
   <212> DNA
   <213> Homo sapiens
<400> 19
   atgaccgcgc cgggcgccgc cgggcgctgc cctcccacga catggctggg ctccctgctg      60
   ttgttggtct gtctcctggc gagcaggagt atcaccgagg aggtgtcgga gtactgtagc     120
   cacatgattg ggagtggaca cctgcagtct ctgcagcggc tgattgacag tcagatggag     180
   acctcgtgcc aaattacatt tgagtttgta gaccaggaac agttgaaaga tccagtgtgc     240
   taccttaaga aggcatttct cctggtacaa gacataatgg aggacaccat gcgcttcaga     300
   gataacaccc ccaatgccat cgccattgtg cagctgcagg aactctcttt gaggctgaag     360
   agctgcttca ccaaggatta tgaagagcat gacaaggcct gcgtccgaac tttctatgag 420
   acacctctcc agttgctgga gaaggtcaag aatgtcttta atgaaacaaa gaatctcctt 480
   gacaaggact ggaatatttt cagcaagaac tgcaacaaca gctttgctga atgctccagc 540
   caagatgtgg tgaccaagcc tgattgcaac tgcctgtacc ccaaagccat ccctagcagt 600
   gacccggcct ctgtctcccc tcatcagccc ctcgccccct ccatggcccc tgtggctggc 660
   ttgacctggg aggactctga gggaactgag ggcagctccc tcttgcctgg tgagcagccc 720
   ctgcacacag tggatccagg cagtgccaag cagcggccac ccaggagcac ctgccagagc 780
   tttgagccgc cagagacccc agttgtcaag gacagcacca tcggtggctc accacagcct 840
   cgcccctctg tcggggcctt caaccccggg atggaggata ttcttgactc tgcaatgggc 900
   actaattggg tcccagaaga agcctctgga gaggccagtg agattcccgt accccaaggg 960
   acagagcttt ccccctccag gccaggaggg ggcagcatgc agacagagcc cgccagaccc 1020
   agcaacttcc tctcagcatc ttctccactc cctgcatcag caaagggcca acagccggca 1080
   gatgtaactg gtaccgcctt gcccagggtg ggccccgtga ggcccactgg ccaggactgg 1140
   aatcacaccc cccagaagac agaccatcca tctgccctgc tcagagaccc cccggagcca 1200
   ggctctccca ggatctcatc actgcgcccc cagggcctca gcaacccctc caccctctct 1260
   gctcagccac agctttccag aagccactcc tcgggcagcg tgctgcccct tggggagctg 1320
   gagggcagga ggagcaccag ggatcggagg agccccgcag agccagaagg aggaccagca 1380
   agtgaagggg cagccaggcc cctgccccgt tttaactccg ttcctttgac tgacacaggc 1440
   catgagaggc agtccgaggg atcctccagc ccgcagctcc aggagtctgt cttccacctg 1500
   ctggtgccca gtgtcatcct ggtcttgctg gccgtcggag gcctcttgtt ctacaggtgg 1560
   aggcggcgga gccatcaaga gcctcagaga gcggattctc ccttggagca accagagggc 1620
   agccccctga ctcaggatga cagacaggtg gaactgccag tgtag 1665
<210> 20
   <211> 1659
   <212> DNA
   <213> Mus musculus
<400> 20
   atgaccgcgc ggggcgccgc ggggcgctgc ccttcttcga catggctggg ctcccggctg      60
   ctgctggtct gtctcctcat gagcaggagt attgccaagg aggtgtcaga acactgtagc     120
   cacatgattg ggaatggaca cctgaaggtc ctgcagcagt tgatcgacag tcaaatggag     180
   acttcatgcc agattgcctt tgaatttgta gaccaggaac agctggatga tcctgtttgc     240
   tacctaaaga aggccttttt tctggtacaa gacataatag atgagaccat gcgctttaaa     300
   gacaacaccc ccaatgctaa cgccaccgag aggctccagg aactctccaa taacctgaac     360
   agctgcttca ccaaggacta tgaggagcag aacaaggcct gtgtccgaac tttccatgag     420
   actcctctcc agctgctgga gaagatcaag aacttcttta atgaaacaaa gaatctcctt     480
   gaaaaggact ggaacatttt taccaagaac tgcaacaaca gctttgctaa gtgctctagc     540
   cgagatgtgg tgaccaagcc tgattgcaac tgcctgtacc ctaaagccac ccctagcagt     600
   gacccggcct ctgcctcccc tcaccagccc cccgccccct ccatggcccc tctggctggc    660
   ttggcttggg atgattctca gaggacagag ggcagctccc tcttgcccag tgagcttccc     720
   cttcgcatag aggacccagg cagtgccaag cagcgaccac ccaggagtac ctgccagacc     780
   ctcgagtcaa cagagcaacc aaaccatggg gacagactca ctgaggactc acaacctcat     840
   ccttctgcgg gggggcccgt ccctggggtg gaagacattc ttgaatcttc actgggcact     900
   aactgggtcc tagaagaagc ttctggagag gctagtgagg gatttttgac ccaggaagca     960
   aagttttccc cctccacgcc tgtagggggc agcatccagg cagagactga cagacccagg    1020
   gccctctcag catctccatt ccctaaatca acagaggacc aaaagccagt ggatataaca    1080
   gacaggccgt tgacagaggt gaaccctatg agacccattg gccagacaca gaataatact    1140
   cctgagaaga ctgatggtac atccacgctg cgtgaagacc accaggagcc aggctctccc    1200
   catattgcga caccgaatcc ccaacgagtc agcaactcag ccacccccgt tgctcagtta   1260
   ctgcttccca aaagccactc ttggggcatt gtgctgcccc ttggggagct tgagggcaag    1320
   agaagtacca gggatcgaag gagccccgca gagctggaag gaggatcagc aagtgagggg    1380
   gcagccaggc ctgtggcccg ttttaattcc attcctttga ctgacacagg ccatgtggag    1440
   cagcatgagg gatcctctga cccccagatc cctgagtctg tcttccacct gctggtgccg    1500
   ggcatcatcc tagtcttgct gactgttggg ggcctcctgt tctacaagtg gaagtggagg    1560
   agccatcgag accctcagac attggattct tctgtggggc gaccagagga cagctccctg    1620
   acccaggatg aggacagaca ggtggaactg ccagtatag                           1659
<210> 21
   <211> 693
   <212> DNA
   <213> Artificial
<220>
   <223> synthetic DNA
<400> 21
   gccaccatgg agacagacac actcctgcta tgggtactgc tgctctgggt tccaggttcc      60
   actggtgacg cggccctcga ggatgcagaa ttccgacatg actcaggata tgaagttcat     120
   catcaaaaat tggtgttctt tgcagaagat gtgggttcaa acaaaggtgc aatcattgga     180
   ctcatggtgg gcggtgttgt catagcgggt acctcttctg gtggtggtgg tatgggtctc     240
   acctcccaac tgcttccccc tctgttcttc ctgctagcat gtgccggcaa ctttgtccac     300
   ggacacaagt gcgatatcac cttacaggag atcatcaaaa ctttgaacag cctcacagag     360
   cagaagactc tgtgcaccga gttgaccgta acagacatct ttgctgcctc caagaacaca     420
   actgagaagg aaaccttctg cagggctgcg actgtgctcc ggcagttcta cagccaccat     480
   gagaaggaca ctcgctgcct gggtgcgact gcacagcagt tccacaggca caagcagctg     540
   atccgattcc tgaaacggct cgacaggaac ctctggggcc tggcgggctt gaattcctgt     600
   cctgtgaagg aagccaacca gagtacgttg gaaaacttct tggaaaggct aaagacgatc     660
   atgagagaga aatattcaaa gtgttcgagc tga                                  693
<210> 22
   <211> 654
   <212> DNA
   <213> Artificial
<220>
   <223> synthetic DNA
<400> 22
   gccaccatgg agacagacac actcctgcta tgggtactgc tgctctgggt tccaggttcc 60
   actggtgacg cggccctcga ggatgcagaa ttccgacatg actcaggata tgaagttcat 120
   catcaaaaat tggtgttctt tgcagaagat gtgggttcaa acaaaggtgc aatcattgga 180
   ctcatggtgg gcggtgttgt catagcgggt acctcttctg gtggtggtgg tatgggtctc 240
   aacccccagc tagttgtcat cctgctcttc tttctcgaat gtaccaggag ccatatccac 300
   ggatgcgaca aaaatcactt gagagagatc atcggcattt tgaacgaggt cacaggagaa 360
   gggacgccat gcacggagat ggatgtgcca aacgtcctca cagcaacgaa gaacaccaca 420
   gagagtgagc tcgtctgtag ggcttccaag gtgcttcgca tattttattt aaaacatggg 480
   aaaactccat gcttgaagaa gaactctagt gttctcatgg agctgcagag actctttcgg 540
   gcttttcgat gcctggattc atcgataagc tgcaccatga atgagtccaa gtccacatca 600
   ctgaaagact tcctggaaag cctaaagagc atcatgcaaa tggattactc gtag 654
<210> 23
   <211> 2527
   <212> DNA
   <213> Artificial
<220>
   <223> synthetic DNA
<400> 23
   gccaccatgg agacagacac actcctgcta tgggtactgc tgctctgggt tccaggttcc 60
   actggtgacg cggccctcga ggatgcagaa ttccgacatg actcaggata tgaagttcat 120
   catcaaaaat tggtgttctt tgcagaagat gtgggttcaa acaaaggtgc aatcattgga 180
   ctcatggtgg gcggtgttgt catagcgtga gtcgacggta ccgcgggccc gggatccgcc 240
   cctctccctc ccccccccct aacgttactg gccgaagccg cttggaataa ggccggtgtg 300
   cgtttgtcta tatgttattt tccaccatat tgccgtcttt tggcaatgtg agggcccgga 360
   aacctggccc tgtcttcttg acgagcattc ctaggggtct ttcccctctc gccaaaggaa 420
   tgcaaggtct gttgaatgtc gtgaaggaag cagttcctct ggaagcttct tgaagacaaa 480
   caacgtctgt agcgaccctt tgcaggcagc ggaacccccc acctggcgac aggtgcctct 540
   gcggccaaaa gccacgtgta taagatacac ctgcaaaggc ggcacaaccc cagtgccacg 600
   ttgtgagttg gatagttgtg gaaagagtca aatggctctc ctcaagcgta ttcaacaagg 660
   ggctgaagga tgcccagaag gtaccccatt gtatgggatc tgatctgggg cctcggtgca 720
   catgctttac atgtgtttag tcgaggttaa aaaaacgtct aggccccccg aaccacgggg 780
   acgtggtttt cctttgaaaa acacgatgat aatatggcca caaccctagt cctgcaggtt 840
   taaacgaatt cgcccttgag gatccaccat gaccgcgcgg ggcgccgcgg ggcgctgccc 900
   ttcttcgaca tggctgggct cccggctgct gctggtctgt ctcctcatga gcaggagtat 960
   tgccaaggag gtgtcagaac actgtagcca catgattggg aatggacacc tgaaggtcct 1020
   gcagcagttg atcgacagtc aaatggagac ttcatgccag attgcctttg aatttgtaga 1080
   ccaggaacag ctggatgatc ctgtttgcta cctaaagaag gccttttttc tggtacaaga 1140
   cataatagat gagaccatgc gctttaaaga caacaccccc aatgctaacg ccaccgagag 1200
   gctccaggaa ctctccaata acctgaacag ctgcttcacc aaggactatg aggagcagaa 1260
   caaggcctgt gtccgaactt tccatgagac tcctctccag ctgctggaga agatcaagaa 1320
   cttctttaat gaaacaaaga atctccttga aaaggactgg aacattttta ccaagaactg 1380
   caacaacagc tttgctaagt gctctagccg agatgtggtg accaagcctg attgcaactg 1440
   cctgtaccct aaagccaccc ctagcagtga cccggcctct gcctcccctc accagccccc 1500
   cgccccctcc atggcccctc tggctggctt ggcttgggat gattctcaga ggacagaggg 1560
   cagctccctc ttgcccagtg agcttcccct tcgcatagag gacccaggca gtgccaagca 1620
   gcgaccaccc aggagtacct gccagaccct cgagtcaaca gagcaaccaa accatgggga 1680
   cagactcact gaggactcac aacctcatcc ttctgcgggg gggcccgtcc ctggggtgga 1740
   agacattctt gaatcttcac tgggcactaa ctgggtccta gaagaagctt ctggagaggc 1800
   tagtgaggga tttttgaccc aggaagcaaa gttttccccc tccacgcctg tagggggcag 1860
   catccaggca gagactgaca gacccagggc cctctcagca tctccattcc ctaaatcaac 1920
   agaggaccaa aagccagtgg atataacaga caggccgttg acagaggtga accctatgag 1980
   acccattggc cagacacaga ataatactcc tgagaagact gatggtacat ccacgctgcg 2040
   tgaagaccac caggagccag gctctcccca tattgcgaca ccgaatcccc aacgagtcag 2100
   caactcagcc acccccgttg ctcagttact gcttcccaaa agccactctt ggggcattgt 2160
   gctgcccctt ggggagcttg agggcaagag aagtaccagg gatcgaagga gccccgcaga 2220
   gctggaagga ggatcagcaa gtgagggggc agccaggcct gtggcccgtt ttaattccat 2280
   tcctttgact gacacaggcc atgtggagca gcatgaggga tcctctgacc cccagatccc 2340
   tgagtctgtc ttccacctgc tggtgccggg catcatccta gtcttgctga ctgttggggg 2400
   cctcctgttc tacaagtgga agtggaggag ccatcgaaac cctcagacat tggattcttc 2460
   tgtggggcga ccagaggaca gctccctgac ccaggatgag gacagacagg tggaactgcc 2520
   agtatag 2527
<210> 24
   <211> 18
   <212> DNA
   <213> Artificial
<220>
   <223> synthetic DNA
<400> 24
   tcttctggtg gtggtggt 18
<210> 25
   <211> 1323
   <212> DNA
   <213> Artificial
<220>
   <223> synthetic DNA
<400> 25
   gccaccatgg aaaccgacac cctgctgctg tgggtgctgc tgctctgggt cccaggatct      60
   acaggcgacg ccgccctcga ggacgccgag ttcagacacg acagcggcta cgaggtgcac     120
   caccagaaac tggtgttctt cgccgaggac gtgggcagca acaagggcgc catcatcggc     180
   ctgatggtcg gcggagtggt cattgccggt accgagccca agagcagcga caagacccac     240
   accagccctc caagccctgc ccctgagctg ctgggcggac ccagcgtgtt cctgttcccc     300
   ccaaagccca aggacaccct gatgatcagc cggacccccg aagtgacctg cgtggtggtg     360
   gacgtgtccc acgaggaccc tgaagtgaag ttcaattggt acgtggacgg cgtggaggtg     420
   cacaacgcca agaccaagcc ccgggaggaa cagtacaaca gcacctaccg ggtggtgtcc     480
   gtgctgaccg tgctgcacca ggactggctg aacggcaaag agtacaagtg caaggtctcc     540
   aacaaggccc tgcctgcccc catcgagaaa accatcagca aggccaaggg ccagcctcgg     600
   gagcctcagg tgtacaccct gccccccagc agggacgagc tgaccaagaa ccaggtgtcc     660
   ctgacctgcc tggtcaaggg cttctacccc agcgatatcg ccgtggagtg ggagagcaac     720
   ggccagcccg agaacaacta caagaccacc ccccctgtgc tggacagcga cggcagcttc     780
   ttcctgtact ccaaactgac cgtggacaag agccggtggc agcagggcaa cgtgttcagc     840
   tgcagcgtga tgcacgaggc cctgcacaac cactacaccc agaagtccct gagcctgagc     900
   cctggcaagg tcgacagcag cggaggcgga ggccacaagt gcgacatcac cctgcaggaa     960
   atcatcaaga ccctgaacag cctgaccgag cagaaaaccc tgtgcaccga gctgaccgtg    1020
   accgacatct tcgccgccag caagaacacc accgagaaag agacattctg cagagccgcc    1080
   accgtgctgc ggcagttcta cagccaccac gagaaggaca ccagatgtct gggcgccacc    1140
   gcccagcagt tccaccggca caagcagctg atccggttcc tgaagcggct ggacagaaat    1200
   ctgtggggcc tggccggcct gaacagctgc cccgtgaaag aggccaacca gagcaccctg    1260
   gaaaactttc tggaacggct caagaccatc atgcgggaga agtacagcaa gtgcagcagc    1320
   tga                                                                  1323
<210> 26
   <211> 1311
   <212> DNA
   <213> Artificial
<220>
   <223> synthetic DNA
<400> 26
   gccaccatgg aaaccgacac cctgctgctg tgggtgctgc tgctctgggt cccaggatct 60
   acaggcgacg ccgccctcga ggacgccgag ttcagacacg acagcggcta cgaggtgcac 120
   caccagaaac tggtgttctt cgccgaggac gtgggcagca acaagggcgc catcatcggc 180
   ctgatggtcg gcggagtggt cattgccggt accgagcccc gggtgcccat cacccagaac 240
   cccagccccc cactgaaaga gagccccccc tctgccgctc ctgatctgct gggcggaccc 300
   agcgtgttca tcttcccacc caagatcaag gacgtgctga tgatcagcct gagccccatg 360
   gtgaccagcg tggtggtgga cgtgtccgag gacgaccccg acgtgcagat cagttggttc 420
   gtgaacaacg tggaggtgca caccgcccag acccagaccc accgggagga ctacaacagc 480
   accctgagag tggtgtccgc cctgcccatc cagcaccagg actggatgag cggcaaagaa 540
   ttcaagagca aagtgaacaa ccgggccctg cccagcccca tcgagaaaac catcagcaag 600
   cccagaggcc ctgtgcgggc tcctcaggtg tacgtgctgc ccccacccgc cgaggaaatg 660
   accaagaaag agttcagcct gaccagcatg atcaccggct ttctgcccgc cgagatcgcc 720
   gtggactgga ccagcaacgg ccggaccgag cagaactaca agaacaccgc caccgtgctg 780
   gacagcgacg gcagctactt catgtacagc aagctgcggg tgcagaagtc cacctgggag 840
   agaggcagcc tgttcgccag cagcgtggtg cacgagggcc tgcacaacca cctgaccacc 900
   aagaccatca gccggtccct gggagtcgac agcagcggag gcggaggcca catccacggc 960
   tgcgacaaga accacctgag agagatcatc ggcatcctga acgaagtgac cggcgagggc 1020
   accccctgta ccgagatgga cgtgcccaac gtgctgaccg ccaccaagaa caccaccgag 1080
   agcgagctgg tgtgccgggc cagcaaggtg ctgcggatct tctacctgaa gcacggcaag 1140
   accccctgcc tgaagaaaaa cagcagcgtg ctgatggaac tgcagcggct gttccgggcc 1200
   ttccggtgcc tggacagcag catcagctgc accatgaacg agagcaagag caccagcctg 1260
   aaggactttc tggaaagcct gaagagtatc atgcagatgg actacagctg a 1311
<210> 27
   <211> 15
   <212> DNA
   <213> Artificial
<220>
   <223> synthetic DNA
<400> 27
   ggtggcggtg gctcg 15
<210> 28
   <211> 1746
   <212> DNA
   <213> Artificial
<220>
   <223> synthetic DNA
<400> 28
   gccaccatgg aaaccgacac cctgctgctg tgggtgctgc tgctctgggt cccaggatct      60
   acaggcgacg ccgccctcga ggacgccgag ttcagacacg acagcggcta cgaggtgcac     120
   caccagaaac tggtgttctt cgccgaggac gtgggcagca acaagggcgc catcatcggc     180
   ctgatggtcg gcggagtggt cattgccggc ggaggcggct ccgatgccga gttccggcac     240
   gattccggct atgaagtcca ccatcagaag ctcgtctttt ttgccgagga tgtggggtct     300
   aacaaagggg ccattattgg gctcatggtc gggggcgtcg tgattgctgg cggcggaggc     360
   agcgacgctg agtttcgcca cgactccgga tacgaagtgc atcaccagaa gctggtcttt     420
   ttcgctgaag atgtcggcag taacaagggg gctattattg gtctgatggt cggcggagtc     480
   gtgatcgcag gggagggggg aagcgacgcc gaattcaggc atgactctgg atatgaggtc     540
   caccaccaga aactcgtgtt ttttgctgag gacgtcggct caaacaaagg cgctatcatt     600
   ggactcatgg tcggaggcgt ggtcattgca ggtaccgagc ccaagagcag cgacaagacc     660
   cacaccagcc ctccaagccc tgcccctgag ctgctgggcg gacccagcgt gttcctgttc     720
   cccccaaagc ccaaggacac cctgatgatc agccggaccc ccgaagtgac ctgcgtggtg     780
   gtggacgtgt cccacgagga ccctgaagtg aagttcaatt ggtacgtgga cggcgtggag     840
   gtgcacaacg ccaagaccaa gccccgggag gaacagtaca acagcaccta ccgggtggtg     900
   tccgtgctga ccgtgctgca ccaggactgg ctgaacggca aagagtacaa gtgcaaggtc     960
   tccaacaagg ccctgcctgc ccccatcgag aaaaccatca gcaaggccaa gggccagcct    1020
   cgggagcctc aggtgtacac cctgcccccc agcagggacg agctgaccaa gaaccaggtg    1080
   tccctgacct gcctggtcaa gggcttctac cccagcgata tcgccgtgga gtgggagagc    1140
   aacggccagc ccgagaacaa ctacaagacc accccccctg tgctggacag cgacggcagc    1200
   ttcttcctgt actccaaact gaccgtggac aagagccggt ggcagcaggg caacgtgttc    1260
   agctgcagcg tgatgcacga ggccctgcac aaccactaca cccagaagtc cctgagcctg 1320
   agccctggca aggtcgacag cagcggaggc ggaggccaca agtgcgacat caccctgcag 1380
   gaaatcatca agaccctgaa cagcctgacc gagcagaaaa ccctgtgcac cgagctgacc 1440
   gtgaccgaca tcttcgccgc cagcaagaac accaccgaga aagagacatt ctgcagagcc 1500
   gccaccgtgc tgcggcagtt ctacagccac cacgagaagg acaccagatg tctgggcgcc 1560
   accgcccagc agttccaccg gcacaagcag ctgatccggt tcctgaagcg gctggacaga 1620
   aatctgtggg gcctggccgg cctgaacagc tgccccgtga aagaggccaa ccagagcacc 1680
   ctggaaaact ttctggaacg gctcaagacc atcatgcggg agaagtacag caagtgcagc 1740
   agctga 1746
<210> 29
   <211> 1734
   <212> DNA
   <213> Artificial
<220>
   <223> synthetic DNA
<400> 29
   gccaccatgg aaaccgacac cctgctgctg tgggtgctgc tgctctgggt cccaggatct      60
   acaggcgacg ccgccctcga ggacgccgag ttcagacacg acagcggcta cgaggtgcac     120
   caccagaaac tggtgttctt cgccgaggac gtgggcagca acaagggcgc catcatcggc     180
   ctgatggtcg gcggagtggt cattgccggc ggaggcggct ccgatgccga gttccggcac     240
   gattccggct atgaagtcca ccatcagaag ctcgtctttt ttgccgagga tgtggggtct     300
   aacaaagggg ccattattgg gctcatggtc gggggcgtcg tgattgctgg cggcggaggc     360
   agcgacgctg agtttcgcca cgactccgga tacgaagtgc atcaccagaa gctggtcttt     420
   ttcgctgaag atgtcggcag taacaagggg gctattattg gtctgatggt cggcggagtc     480
   gtgatcgcag ggggaggggg aagcgacgcc gaattcaggc atgactctgg atatgaggtc     540
   caccaccaga aactcgtgtt ttttgctgag gacgtcggct caaacaaagg cgctatcatt     600
   ggactcatgg tcggaggcgt ggtcattgca ggtaccgagc cccgggtgcc catcacccag     660
   aaccccagcc ccccactgaa agagagcccc ccctctgccg ctcctgatct gctgggcgga     720
   cccagcgtgt tcatcttccc acccaagatc aaggacgtgc tgatgatcag cctgagcccc     780
   atggtgacca gcgtggtggt ggacgtgtcc gaggacgacc ccgacgtgca gatcagttgg     840
   ttcgtgaaca acgtggaggt gcacaccgcc cagacccaga cccaccggga ggactacaac     900
   agcaccctga gagtggtgtc cgccctgccc atccagcacc aggactggat gagcggcaaa     960
   gaattcaaga gcaaagtgaa caaccgggcc ctgcccagcc ccatcgagaa aaccatcagc    1020
   aagcccagag gccctgtgcg ggctcctcag gtgtacgtgc tgcccccacc cgccgaggaa    1080
   atgaccaaga aagagttcag cctgaccagc atgatcaccg gctttctgcc cgccgagatc    1140
   gccgtggact ggaccagcaa cggccggacc gagcagaact acaagaacac cgccaccgtg    1200
   ctggacagcg acggcagcta cttcatgtac agcaagctgc gggtgcagaa gtccacctgg    1260
   gagagaggca gcctgttcgc cagcagcgtg gtgcacgagg gcctgcacaa ccacctgacc    1320
   accaagacca tcagccggtc cctgggagtc gacagcagcg gaggcggagg ccacatccac    1380
   ggctgcgaca agaaccacct gagagagatc atcggcatcc tgaacgaagt gaccggcgag    1440
   ggcaccccct gtaccgagat ggacgtgccc aacgtgctga ccgccaccaa gaacaccacc    1500
   gagagcgagc tggtgtgccg ggccagcaag gtgctgcgga tcttctacct gaagcacggc    1560
   aagaccccct gcctgaagaa aaacagcagc gtgctgatgg aactgcagcg gctgttccgg    1620
   gccttccggt gcctggacag cagcatcagc tgcaccatga acgagagcaa gagcaccagc    1680
   ctgaaggact ttctggaaag cctgaagagt atcatgcaga tggactacag ctga          1734
<210> 30
   <211> 1335
   <212> DNA
   <213> Artificial
<220>
   <223> synthetic DNA
<400> 30
   gccaccatgg aaaccgacac cctgctgctg tgggtgctgc tgctctgggt cccaggatct 60
   acaggcgacg ccgccctcga ggacgccgag ttcagacacg acagcggcta cgaggtgcac 120
   caccagaaac tggtgttctt cgccgaggac gtgggcagca acaagggcgc catcatcggc 180
   ctgatggtcg gcggagtggt cattgccggc ggaggcggct ccgatgccga gttccggcac 240
   gattccggct atgaagtcca ccatcagaag ctcgtctttt ttgccgagga tgtggggtct 300
   aacaaagggg ccattattgg gctcatggtc gggggcgtcg tgattgctgg cggcggaggc 360
   agcgacgctg agtttcgcca cgactccgga tacgaagtgc atcaccagaa gctggtcttt 420
   ttcgctgaag atgtcggcag taacaagggg gctattattg gtctgatggt cggcggagtc 480
   gtgatcgcag gggagggggg aagcgacgcc gaattcaggc atgactctgg atatgaggtc 540
   caccaccaga aactcgtgtt ttttgctgag gacgtcggct caaacaaagg cgctatcatt 600
   ggactcatgg tcggaggcgt ggtcattgca ggtaccgagc ccaagagcag cgacaagacc 660
   cacaccagcc ctccaagccc tgcccctgag ctgctgggcg gacccagcgt gttcctgttc 720
   cccccaaagc ccaaggacac cctgatgatc agccggaccc ccgaagtgac ctgcgtggtg 780
   gtggacgtgt cccacgagga ccctgaagtg aagttcaatt ggtacgtgga cggcgtggag 840
   gtgcacaacg ccaagaccaa gccccgggag gaacagtaca acagcaccta ccgggtggtg 900
   tccgtgctga ccgtgctgca ccaggactgg ctgaacggca aagagtacaa gtgcaaggtc 960
   tccaacaagg ccctgcctgc ccccatcgag aaaaccatca gcaaggccaa gggccagcct 1020
   cgggagcctc aggtgtacac cctgcccccc agcagggacg agctgaccaa gaaccaggtg 1080
   tccctgacct gcctggtcaa gggcttctac cccagcgata tcgccgtgga gtgggagagc 1140
   aacggccagc ccgagaacaa ctacaagacc accccccctg tgctggacag cgacggcagc 1200
   ttcttcctgt actccaaact gaccgtggac aagagccggt ggcagcaggg caacgtgttc 1260
   agctgcagcg tgatgcacga ggccctgcac aaccactaca cccagaagtc cctgagcctg 1320
   agccctggca agtga 1335
<210> 31
   <211> 1350
   <212> DNA
   <213> Artificial
<220>
   <223> synthetic DNA
<400> 31
   gccaccatgg aaaccgacac cctgctgctg tgggtgctgc tgctctgggt cccaggatct      60
   acaggcgacg ccgccctcga ggacgccgag ttcagacacg acagcggcta cgaggtgcac     120
   caccagaaac tggtgttctt cgccgaggac gtgggcagca acaagggcgc catcatcggc     180
   ctgatggtcg gcggagtggt cattgccggc ggaggcggct ccgatgccga gttccggcac     240
   gattccggct atgaagtcca ccatcagaag ctcgtctttt ttgccgagga tgtggggtct     300
   aacaaagggg ccattattgg gctcatggtc gggggcgtcg tgattgctgg cggcggaggc     360
   agcgacgctg agtttcgcca cgactccgga tacgaagtgc atcaccagaa gctggtcttt     420
   ttcgctgaag atgtcggcag taacaagggg gctattattg gtctgatggt cggcggagtc     480
   gtgatcgcag ggggaggggg aagcgacgcc gaattcaggc atgactctgg atatgaggtc      540
   caccaccaga aactcgtgtt ttttgctgag gacgtcggct caaacaaagg cgctatcatt     600
   ggactcatgg tcggaggcgt ggtcattgca ggtaccgagc cccgggtgcc catcacccag     660
   aaccccagcc ccccactgaa agagagcccc ccctctgccg ctcctgatct gctgggcgga     720
   cccagcgtgt tcatcttccc acccaagatc aaggacgtgc tgatgatcag cctgagcccc     780
   atggtgacca gcgtggtggt ggacgtgtcc gaggacgacc ccgacgtgca gatcagttgg     840
   ttcgtgaaca acgtggaggt gcacaccgcc cagacccaga cccaccggga ggactacaac     900
   agcaccctga gagtggtgtc cgccctgccc atccagcacc aggactggat gagcggcaaa     960
   gaattcaaga gcaaagtgaa caaccgggcc ctgcccagcc ccatcgagaa aaccatcagc    1020
   aagcccagag gccctgtgcg ggctcctcag gtgtacgtgc tgcccccacc cgccgaggaa    1080
   atgaccaaga aagagttcag cctgaccagc atgatcaccg gctttctgcc cgccgagatc    1140
   gccgtggact ggaccagcaa cggccggacc gagcagaact acaagaacac cgccaccgtg    1200
   ctggacagcg acggcagcta cttcatgtac agcaagctgc gggtgcagaa gtccacctgg    1260
   gagagaggca gcctgttcgc cagcagcgtg gtgcacgagg gcctgcacaa ccacctgacc    1320
   accaagacca tcagccggtc cctgggatga                                    1350
<210> 32
   <211> 696
   <212> DNA
   <213> Homo sapiens
<400> 32
   gagcccaaga gcagcgacaa gacccacacc agccctccaa gccctgcccc tgagctgctg 60
   ggcggaccca gcgtgttcct gttcccccca aagcccaagg acaccctgat gatcagccgg 120
   acccccgaag tgacctgcgt ggtggtggac gtgtcccacg aggaccctga agtgaagttc 180
   aattggtacg tggacggcgt ggaggtgcac aacgccaaga ccaagccccg ggaggaacag 240
   tacaacagca cctaccgggt ggtgtccgtg ctgaccgtgc tgcaccagga ctggctgaac 300
   ggcaaagagt acaagtgcaa ggtctccaac aaggccctgc ctgcccccat cgagaaaacc 360
   atcagcaagg ccaagggcca gcctcgggag cctcaggtgt acaccctgcc ccccagcagg 420
   gacgagctga ccaagaacca ggtgtccctg acctgcctgg tcaagggctt ctaccccagc 480
   gatatcgccg tggagtggga gagcaacggc cagcccgaga acaactacaa gaccaccccc 540
   cctgtgctgg acagcgacgg cagcttcttc ctgtactcca aactgaccgt ggacaagagc 600
   cggtggcagc agggcaacgt gttcagctgc agcgtgatgc acgaggccct gcacaaccac 660
   tacacccaga agtccctgag cctgagccct ggcaag 696
<210> 33
   <211> 711
   <212> DNA
   <213> Mus musculus
<400> 33
   gagccccggg tgcccatcac ccagaacccc agccccccac tgaaagagag ccccccctct 60
   gccgctcctg atctgctggg cggacccagc gtgttcatct tcccacccaa gatcaaggac 120
   gtgctgatga tcagcctgag ccccatggtg accagcgtgg tggtggacgt gtccgaggac 180
   gaccccgacg tgcagatcag ttggttcgtg aacaacgtgg aggtgcacac cgcccagacc 240
   cagacccacc gggaggacta caacagcacc ctgagagtgg tgtccgccct gcccatccag 300
   caccaggact ggatgagcgg caaagaattc aagagcaaag tgaacaaccg ggccctgccc 360
   agccccatcg agaaaaccat cagcaagccc agaggccctg tgcgggctcc tcaggtgtac 420
   gtgctgcccc cacccgccga ggaaatgacc aagaaagagt tcagcctgac cagcatgatc 480
   accggctttc tgcccgccga gatcgccgtg gactggacca gcaacggccg gaccgagcag 540
   aactacaaga acaccgccac cgtgctggac agcgacggca gctacttcat gtacagcaag 600
   ctgcgggtgc agaagtccac ctgggagaga ggcagcctgt tcgccagcag cgtggtgcac 660
   gagggcctgc acaaccacct gaccaccaag accatcagcc ggtccctggg a 711